(19) Europäisches Patentamt
European Patent Office
Office européen des brevets

(11) Veröffentlichungsnummer : **0 110 048**
**B1**

# (12) EUROPÄISCHE PATENTSCHRIFT

(45) Veröffentlichungstag der Patentschrift : **03.12.86**

(21) Anmeldenummer : **83109545.0**

(22) Anmeldetag : **26.09.83**

(51) Int. Cl.⁴ : **C 07 D249/08**, C 07 D405/10, A 01 N 43/64// C07D317/26, C07D407/10

## (54) Substituierte 1-Hydroxyethyl-triazolyl-Derivate.

(30) Priorität : **08.10.82 DE 3237400**

(43) Veröffentlichungstag der Anmeldung : **13.06.84 Patentblatt 84/24**

(45) Bekanntmachung des Hinweises auf die Patenterteilung : **03.12.86 Patentblatt 86/49**

(84) Benannte Vertragsstaaten : **AT BE CH DE FR GB IT LI NL SE**

(56) Entgegenhaltungen :
**EP-A- 0 052 424**
**EP-A- 0 061 835**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Holmwood, Graham, Dr.**
**Krutscheider Weg 105**
**D-5600 Wuppertal 11 (DE)**
Erfinder : **Stetter, Jörg, Dr.**
**Gellertweg 4**
**D-5600 Wuppertal 1 (DE)**
Erfinder : **Büchel, Karl Heinz, Prof. Dr.**
**Dabringhausener Strasse 42**
**D-5093 Burscheid (DE)**
Erfinder : **Reinecke, Paul, Dr.**
**Lessingstrasse 11**
**D-5090 Leverkusen 3 (DE)**
Erfinder : **Brandes, Wilhelm, Dr.**
**Eichendorffstrasse 3**
**D-5653 Leichlingen (DE)**
Erfinder : **Scheinpflug, Hans, Dr.**
**Am Thelenhof 15**
**D-5090 Leverkusen 1 (DE)**

EP 0 110 048 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue substituierte 1-Hydroxyethyl-triazolyl-Derivate, mehrere Verfahren zu ihrer Herstellung sowie ihre Verwendung als Fungizide.

Es ist bereits bekannt geworden, daß 3,3-Dimethyl-1-phenoxy-2-(1,2,4-triazol-1-yl-methyl)-2-butanole gute fungizide Eigenschaften aufweisen (vgl. DE-A 3 018 866). Die Wirkung dieser Verbindungen ist jedoch, insbesondere bei niedrigen Aufwandmengen und -konzentrationen nicht immer voll befriedigend.

Ferner sind aus der EP-A 0 052 424 und der EP-A 0 061 835 bestimmte 1-Hydroxyethyl-triazolyl-Derivate mit fungizider Wirkung bekannt. Es werden jedoch keine derartigen Stoffe beschrieben, in denen der Phenyl-Rest durch Formyl-, Acyl-, Acetalketal- oder Oxim-Gruppen substituiert ist.

Es wurden neue substituierte 1-Hydroxyethyl-triazolyl-Derivate der allgemeinen Formel

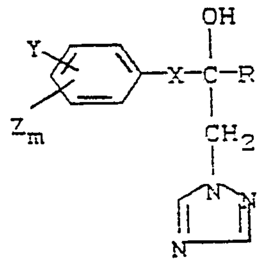

(I)

in welcher

R für geradkettiges oder verzweigtes Alkyl mit 1 bis 4 Kohlenstoffatomen, für gegebenenfalls einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen und Alkoxy mit 1 bis 2 Kohlenstoffatomen, ferner für gegebenenfalls einfach oder mehrfach, gleich oder verschieden substituiertes Phenyl steht, wobei als Substituenten die bei $R^3$ genannten Phenylsubstituenten infrage kommen, sowie für die Gruppierungen

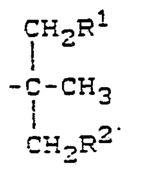

und

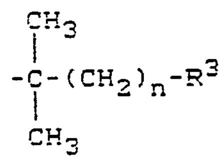

steht ;

$R^1$ für Wasserstoff oder Halogen steht ;

$R^2$ für Halogen steht ;

$R^3$ für Alkyl, Alkoxy und Alkylthio mit jeweils 1 bis 4 Kohlenstoffatomen, für Halogenalkoxy und Halogenalkylthio mit jeweils 1 bis 2 Kohlenstoffatomen und 1 bis 5 gleichen oder verschiedenen Halogenatomen steht, ferner für Alkenyl mit 2 bis 6 Kohlenstoffatomen, für Alkoxycarbonyl mit 1 bis 4 Kohlenstoffatomen im Alkylteil, für Cyano sowie für jeweils gegebenenfalls einfach oder mehrfach substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil, steht, wobei jeweils als Phenylsubstituenten genannt seien : Halogen, Alkyl mit 1 bis 4 Kohlenstoffatomen ; Alkoxy und Alkylthio mit jeweils 1 bis 2 Kohlenstoffatomen ; Halogenalkyl, Halogenalkoxy und Halogenalkylthio mit jeweils 1 bis 2 Kohlenstoff- und 1 bis 5 gleichen oder verschiedenen Halogenatomen, Cyclohexyl, Dialkylamino mit 1 bis 4 Kohlenstoffatomen in jedem Alkylteil, Nitro, Cyano, Alkoxycarbonyl mit 1 bis 4 Kohlenstoffatomen im Alkylteil, sowie gegebenenfalls durch Halogen substituiertes Phenyl,

n für die Zahlen 0, 1 oder 2 steht ;

X für die Gruppierungen $-OCH_2-$, $-SCH_2-$, $-(CH_2)_p$ —oder $-CH=CH-$ steht ;

Y für die Gruppierungen $-CO-Y^1$, $-C(OR^4)_2-Y^1$ und

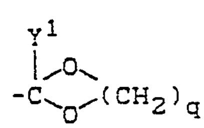

sowie auch für die Gruppierung $-C(Y^1)=N-OY^2$ steht ;

$Y^1$ für Wasserstoff, für Alkyl mit 1 bis 4 Kohlenstoffatomen, für Alkenyl und Alkinyl mit jeweils 2 bis 4 Kohlenstoffatomen, für gegebenenfalls einfach oder mehrfach, gleich oder verschieden substituiertes Cycloalkyl mit 3 bis 7 Kohlenstoffatomen steht, wobei als Substituenten genannt seien : Halogen, Alkyl

mit 1 bis 4 Kohlenstoffatomen und Alkoxy mit 1 bis 2 Kohlenstoffatomen, sowie für jeweils gegebenenfalls einfach oder mehrfach, gleich oder verschieden substituiertes Phenyl oder Benzyl steht, wobei als Substituenten die bei $R^3$ genannten Phenylsubstituenten infrage kommen ;

$Y^2$ für Wasserstoff, für geradkettiges oder verzweigtes Alkyl mit 1 bis 4 Kohlenstoffatomen, für Alkenyl und Alkinyl mit jeweils 2 bis 6 Kohlenstoffatomen, für gegebenenfalls einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen und Alkoxy mit 1 bis 2 Kohlenstoffatomen ; sowie für gegebenenfalls einfach oder mehrfach, gleich oder verschieden substituiertes Benzyl steht, wobei als Substituenten die bei $R^3$ genannten Phenylsubstituenten infrage kommen ;

$R^4$ für Alkyl mit 1 bis 4 Kohlenstoffatomen steht ;

q für die Zahlen 2 oder 3 steht ;

Z für Halogen, für Alkyl, Alkoxy und Alkylthio mit jeweils 1 bis 4 Kohlenstoffatomen, sowie für Halogenalkyl, Halogenalkoxy oder Halogenalkylthio mit jeweils 1 bis 2 Kohlenstoffatomen und 1 bis 5 gleichen oder verschiedenen Halogenatomen steht ;

m für die Zahlen 0, 1 oder 2 steht und

p für die Zahlen 0, 1 oder 2 steht ;

sowie deren Säureadditions-Salze und Metallsalz-Komplexe gefunden.

Die Verbindungen der Formel (I) besitzen ein asymmetrisches Kohlenstoffatom und können deshalb in den beiden optischen Isomerenformen anfallen. Außerdem können die Verbindungen der Formel (I) gegebenenfalls in verschiedenen geometrischen Isomeren vorkommen, je nach Bedeutung der Substituenten X und Y.

Weiterhin wurde gefunden, daß man die substituierten 1-Hydroxyethyl-triazolyl-Derivate der Formel (I) sowie deren Säureadditions-Salze und Metallsalz-Komplexe erhält, wenn man

a) Oxirane der Formel

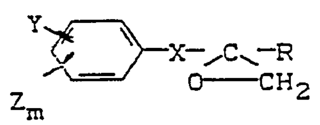

(II)

in welcher R, X, Y, Z und m die oben angegebene Bedeutung haben, mit 1,2,4-Triazol der Formel

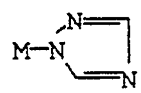

(III)

in welcher M für Wasserstoff oder ein Alkalimetall steht, in Gegenwart eines Verdünnungsmittels und gegebenenfalls in Gegenwart einer Base umsetzt, oder

b) Triazolylmethyl-oxirane der Formel

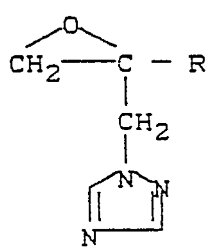

(IV)

in welcher R die oben angegebene Bedeutung hat, mit (Thio)Phenolen der Formel

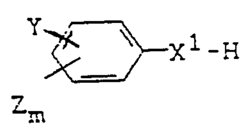

(V)

in welcher

Y, Z und m die oben angegebene Bedeutung haben und

$X^1$ für Sauerstoff oder Schwefel steht,

in Gegenwart eines Verdünnungsmittels und gegebenenfalls in Gegenwart einer Base umsetzt, oder

c) die nach den Verfahren (a) und (b) erhältlichen erfindungsgemäßen Verbindungen der Formel

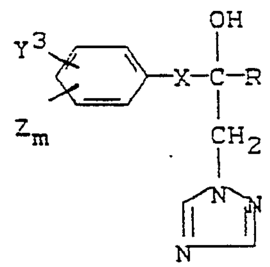

(Ia)

in welcher

R, X, Z und m die oben angegebene Bedeutung haben und

$Y^3$ für die Gruppierungen $—CO—Y^1$, $—C(OR^4)_2Y^1$ und

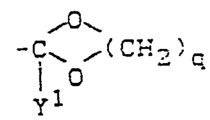

steht, mit Hydroxylamin-Derivaten der Formel

$$H_2N—O—Y^2 \quad (VI)$$

in welcher $Y^2$ die oben angegebene Bedeutung hat, in Gegenwart eines Verdünnungsmittels umsetzt, oder

d) die nach den Verfahren a), b) und c) erhältlichen erfindungsgemäßen Verbindungen der Formel

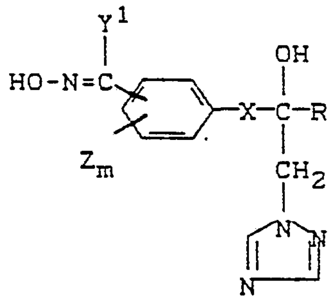

(Ib)

in welcher R, X, $Y^1$, Z und m die oben angegebene Bedeutung haben, mit Halogeniden der Formel

$$Y^4—Hal \quad (VII)$$

in welcher

Hal für Chlor, Brom oder lod steht und

$Y^4$ für die Bedeutungen von $Y^2$, außer für Wasserstoff, steht,

in Gegenwart eines Verdünnungsmittels und gegebenenfalls in Gegenwart einer Base umsetzt, und gegebenenfalls an die nach obigen Verfahren erhaltenen Verbindungen der Formel (I) anschließend eine Säure oder ein Metallsalz addiert.

Außerdem wurde gefunden, daß die neuen substituierten 1-Hydroxyethyl-triazolyl-Derivate der Formel (I) starke fungizide Eigenschaften aufweisen.

Überraschenderweise zeigen die erfindungsgemäßen substituierten 1-Hydroxyethyl-triazolyl-Derivate der Formel (I) bessere fungizide Wirkungen, als die obengenannten, aus dem Stand der Technik bekannten 3,3-Dimethyl-1-phenoxy-2-(1,2,4-triazol-1-yl-methyl)-2-butanole, welche konstitutionell und wirkungsmäßig naheliegende Verbindungen sind. Die erfindungsgemäßen Wirkstoffe stellen somit eine Bereicherung der Technik dar.

Die erfindungsgemäßen substituierten 1-Hydroxyethyl-triazolyl-Derivate sind durch die Formel (I) allgemein definiert.

Bevorzugt sind diejenigen Verbindungen der Formel (I), in denen

R für tert.-Butyl oder Isopropyl steht, für jeweils gegebenenfalls einfach bis dreifach, gleich oder verschieden substituiertes Cyclopropyl, Cyclopentyl oder Cyclohexyl steht, wobei als Substituenten genannt seien : Methyl, Ethyl, Isopropyl, Methoxy und Ethoxy, ferner für gegebenenfalls einfach bis dreifach, gleich oder verschieden substituiertes Phenyl steht, wobei als Substituenten genannt seien : Fluor, Chlor, Methyl, Trifluormethyl, Phenyl und Chlorphenyl, sowie für die Gruppierungen

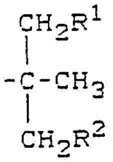 und 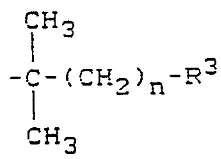

steht, wobei

$R^1$ für Wasserstoff, Fluor oder Chlor steht ;

$R^2$ für Fluor oder Chlor steht ;

$R^3$ für Methyl, Ethyl, Propyl, Methoxy, Ethoxy, Methylthio, Ethylthio, Trifluormethoxy, Trifluormethylthio, Vinyl, Methoxycarbonyl, Ethoxycarbonyl, Cyano sowie für jeweils gegebenenfalls einfach bis zweifach, gleich oder verschieden substituiertes Phenyl, Phenoxy, Phenylthio, Phenylmethoxy oder Phenylmethylthio steht, wobei jeweils als Phenylsubstituenten genannt seien : Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl ; und

n für die Zahlen 0, 1 oder 2 steht ;

X für die Gruppierungen $—OCH_2—$, $—SCH_2—$, $—(CH_2)_p—$ oder $—CH = CH—$ steht ;

Y für die Gruppierungen $—CO—Y^1$, $—C(OR^4)_2—Y^1$ und

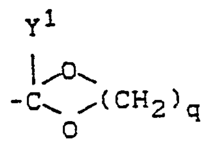

sowie auch für die Gruppierung $—C(Y^1) = N—OY^2$ steht ; wobei

$Y^1$ für Wasserstoff, Methyl, Ethyl, Isopropyl oder für gegebenenfalls einfach bis zweifach, gleich oder verschieden durch Fluor, Chlor, Methyl oder Trifluormethyl substituiertes Phenyl steht ;

$Y^2$ für Wasserstoff, Methyl,. Ethyl, n-Propyl, n-Butyl, Allyl, Propargyl oder für gegebenenfalls einfach bis zweifach, gleich oder verschieden durch Fluor, Chlor, Methyl, Trifluormethyl oder Trifluormethoxy substituiertes Benzyl steht ;

$R^4$ für Methyl, Ethyl oder Propyl steht ; und

q für die Zahlen 2 oder 3 steht ;

Z für Fluor, Chlor, Brom, Methyl, Methoxy, Methylthio, Trifluormethyl, Trifluormethoxy oder Trifluormethylthio steht, und

m für die Zahlen 0, 1 oder 2 steht ; und

p für die Zahlen 0, 1 oder 2 steht.

Bevorzugte erfindungsgemäße Verbindungen sind auch Additionsprodukte aus Säuren und denjenigen substituierten 1-Hydroxyethyl-triazolyl-Derivaten der Formel (I), in denen die Substituenten R, X, Y und $Z_m$ die Bedeutungen haben, die bereits für diese Substituenten als bevorzugt genannt wurden.

Zu den Säuren die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z. B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten 1-Hydroxyethyl-triazolyl-Derivaten der Formel (I), in denen die Substituenten X, Y und $Z_m$ die Bedeutungen haben, die bereits für diese Substituenten als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, ferner Phosphorsäure, Salpetersäure und Schwefelsäure.

Verwendet man beispielsweise 2-{2-[4-(1,3-dioxolan-2-yl)-phenyl]-ethenyl}-2-tert.-butyl-oxiran und 1,2,4-Triazolnatrium als Ausgangsstoffe, so kann der Ablauf des erfindungsgemäßen Verfahrens (a) durch das folgende Formelschema wiedergegeben werden :

(Siehe Formelschema Seite 6 f.)

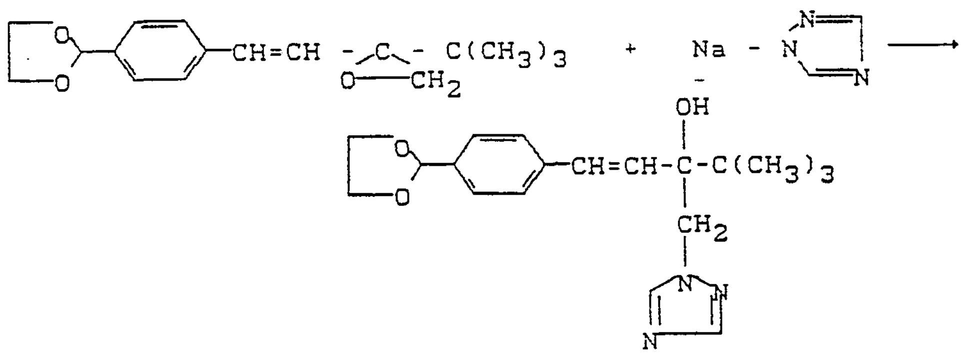

Verwendet man beispielsweise 2-tert.-Butyl-2-(1,2,4-triazol-1-yl-methyl)-oxiran und p-Hydroxyacetophenon als Ausgangsstoffe, so kann der Ablauf des erfindungsgemäßen Verfahrens (b) durch das folgende Formelschema wiedergegeben werden :

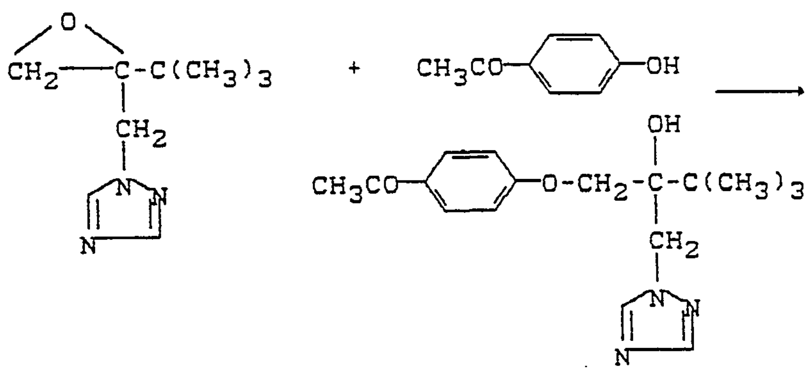

Verwendet man beispielsweise 2-(4-Acetophenoxymethyl)-3,3-dimethyl-1-(1,2,4-triazol-1-yl)-2-butanol und Hydroxylamin-hydrochlorid als Ausgangsstoffe , so kann der Ablauf des erfindungsgemäßen Verfahrens (c) durch das folgende Formelschema wiedergegeben werden :

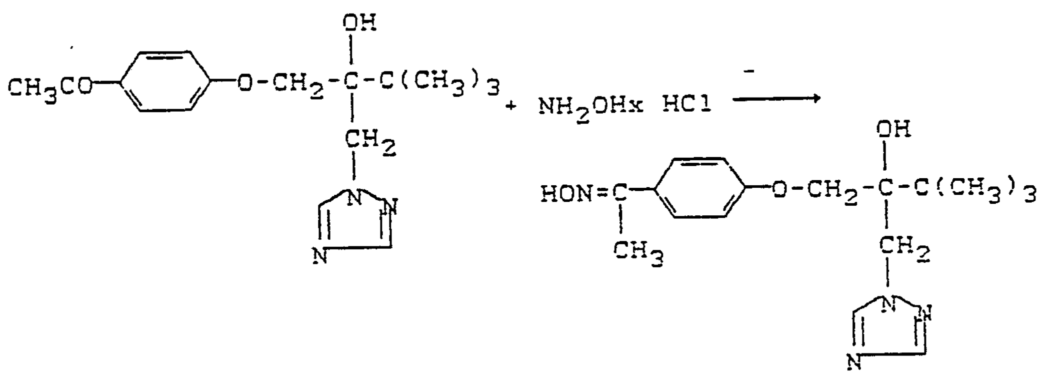

Verwendet man beispielsweise 3,3-Dimethyl-2-[4-(1-hydroximinoethyl)-phenoxymethyl]-1-(1,2,4-triazol-1-yl)-2-butanol und 2,4-Dichlorbenzylchlorid als Ausgangsstoffe, so kann der Ablauf des erfindungsgemäßen Verfahrens (d) durch das folgende Formelschema wiedergegeben werden :

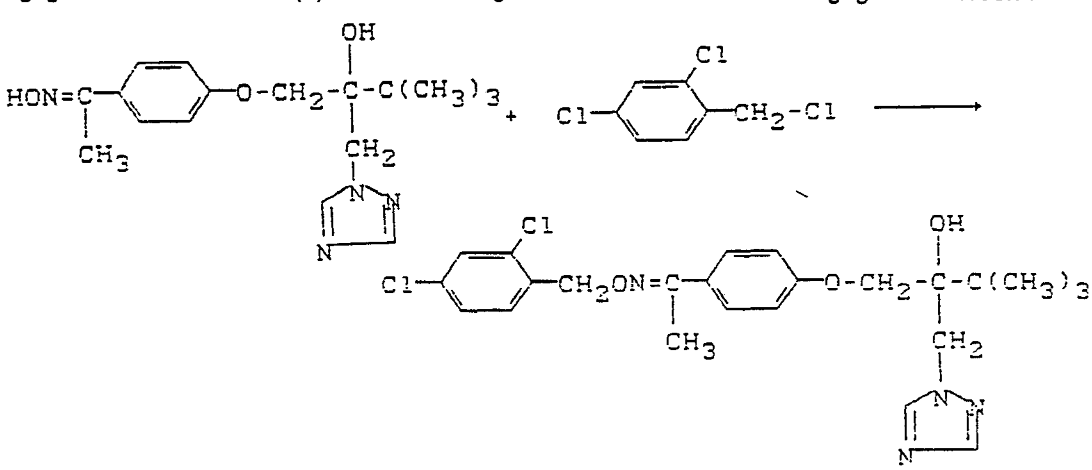

Die bei der Durchführung des erfindungsgemäßen Verfahrens (a) als Ausgangsstoffe zu verwendenden Oxirane sind durch die Formel (II) allgemein definiert. In dieser Formel haben R, X, Y, Z und der Index m bevorzugt die Bedeutungen, die bereits im Zusammenhang mit der Beschreibung der erfindungsgemäßen Stoffe der Formel (I) für diese Substituenten bzw. für den Index m als bevorzugt genannt wurden.

Die Oxirane der Formel (II) sind noch nicht bekannt. Sie stellen interessante Zwischenprodukte dar und können in allgemein bekannter Art und Weise erhalten werden, indem man Ketone der Formel

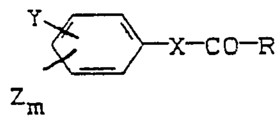
(VIII)

in welcher R, X, Y, Z und m die oben angegebene Bedeutung haben, entweder

α) mit Dimethyloxosulfonium-methylid der Formel

$$\overset{\delta+}{(CH_3)_2SO}\overset{\delta-}{CH_2} \qquad (IX)$$

in Gegenwart eines Verdünnungsmittels umsetzt, oder

β) mit Trimethylsulfonium-methylsulfat der Formel

$$\left[(CH_3)_3\overset{\oplus}{S}\right] \quad (CH_3SO_4)^{\ominus} \qquad (X)$$

in Gegenwart eines inerten organischen Lösungsmittels sowie in Gegenwart einer Base umsetzt.

Die bei der Herstellung der Oxirane der Formel (II) als Ausgangsstoffe benötigten Ketone der Formel (VIII) lassen sich nach im Prinzip bekannten Verfahren herstellen (vgl. z. B. die Herstellungsbeispiele).

Das bei der Verfahrensvariante (α) benötigte Dimethyloxosulfoniummethylid der Formel (VII) ist bekannt (vgl. J. Amer. Chem. Soc. *87*, 1363-1364 (1965)). Es wird bei der obigen Umsetzung in frisch zubereitetem Zustand verarbeitet, indem man es *in situ* durch Umsetzung von Trimethyloxosulfoniumiodid mit Natriumhydrid, Natriumamid bzw. Kalium-tert.-butylat in Gegenwart eines Verdünnungsmittels erzeugt.

Das bei der Verfahrensvariante (β) benötigte Trimethyl-sulfonium-methylsulfat der Formel (VIII) ist ebenfalls bekannt (vgl. Heterocyclus *8*, 397 (1977)). Es wird bei der obigen Umsetzung ebenfalls in frisch hergestelltem Zustand eingesetzt, indem man es durch Reaktion von Dimethylsulfid mit Dimethylsulfat *in situ* erzeugt.

Bei der Variante (α) des Verfahrens zur Herstellung der Oxirane der Formel (II) kommt als Verdünnungsmittel vorzugsweise Dimethylsulfoxid infrage.

Die Reaktionstemperaturen können bei der oben beschriebenen Verfahrensvariante (α) innerhalb eines größeren Bereiches variiert werden. Im allgemeinen arbeitet man bei Temperaturen zwischen 20 °C und 80 °C.

Die Durchführung des Verfahrens zur Herstellung der Oxirane der Formel (II) nach der Variante (α) und die Aufarbeitung des bei dieser Synthese anfallenden Reaktionsgemisches erfolgen nach üblichen Methoden (vgl. J. Amer. Chem. Soc. *87*, 1363-1364 (1965)).

Bei der Variante (β) zur Herstellung der Oxirane der Formel (II) kommt als inertes organisches Lösungsmittel vorzugsweise Acetonitril in Betracht.

Als Base können bei der Verfahrensvariante (β) starke anorganische oder organische Basen verwendet werden. Vorzugsweise infrage kommt Natriummethylat.

Die Reaktionstemperaturen können bei der oben beschriebenen Verfahrensvariante (β) innerhalb eines bestimmten Bereiches variiert werden. Im allgemeinen arbeitet man bei Temperaturen zwischen 0 °C und 60 °C, vorzugsweise bei Raumtemperatur.

Die Durchführung des Verfahrens zur Herstellung der Oxirane der Formel (II) nach der Variante (β) und die Aufarbeitung des bei dieser Synthese anfallenden Reaktionsproduktes erfolgt nach üblichen Methoden (vgl. Heterocycles *8*, 397 (1977)).

Die Oxirane der Formel (II) können bei dem erfindungsgemäßen Verfahren gegebenenfalls ohne Isolierung direkt weiter umgesetzt werden.

Die außerdem für das erfindungsgemäße Verfahren (a) als Ausgangsstoffe zu verwendenden 1,2,4-Triazole sind durch die Formel (III) allgemein definiert. In dieser Formel steht M vorzugsweise für Wasserstoff, Natrium oder Kalium.

Die 1,2,4-Triazole der Formel (III) sind allgemein bekannte Verbindungen der organischen Chemie.

Die bei der Durchführung des erfindungsgemäßen Verfahrens (b) als Ausgangsstoffe zu verwendenden Triazolylmethyl-oxirane sind durch die Formel (IV) allgemein definiert. In dieser Formel hat R bevorzugt die Bedeutungen, die bereits im Zusammenhang mit der Beschreibung der erfindungsgemäßen Stoffe der Formel (I) für diesen Substituenten als bevorzugt genannt wurden.

Die Triazolylmethyl-oxirane der Formel (IV) sind bekannt (vgl. DE-A 3 111 238 und EP-A 0 044 605), bzw. sind sie Gegenstand der DE-A 3 202 601 bzw. können sie in allgemein bekannter Art und Weise erhalten werden, indem man Triazolyl-ketone der Formel

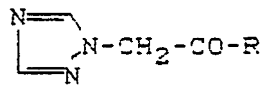

(XI)

in welcher R die oben angegebene Bedeutung hat, entsprechend den oben angegebenen Verfahrensvarianten (α) und (β) epoxidiert.

Die Triazolyl-ketone der Formel (XI) sind bekannt (vgl. DE-A 2 431 407, DE-A 2 638 470, DE-A 2 820 361 und DE-A 3 048 266) bzw. lassen sie sich nach im Prinzip bekannten Verfahren herstellen.

Die außerdem für das erfindungsgemäße Verfahren (b) als Ausgangsstoffe zu verwendenden (Thio)Phenole sind durch die Formel (V) allgemein definiert. In dieser Formel stehen Y, Z und der Index m bevorzugt für die Bedeutungen, die bereits im Zusammenhang mit der Beschreibung der erfindungsgemäßen Stoffe der Formel (I) für diese Substituenten bzw. für den Index m als bevorzugt genannt wurden. $X^1$ steht für Sauerstoff oder Schwefel.

Die (Thio)Phenole der Formel (V) sind bekannt, bzw. sind sie Gegenstand einer eigenen älteren Anmeldung (vgl. DE-A 3 132 335), bzw. können sie in allgemein üblicher Art und Weise erhalten werden.

Die bei der Durchführung des erfindungsgemäßen Verfahrens (c) als Ausgangsstoffe zu verwendenden Verbindungen der Formel (Ia) sind erfindungsgemäße Stoffe.

Die außerdem für das erfindungsgemäße Verfahren (c) als Ausgangsstoffe zu verwendenden Hydroxylamin-Derivate sind durch die Formel (VI) allgemein definiert. In dieser Formel steht $Y^2$ bevorzugt für die Bedeutungen, die bereits im Zusammenhang mit der Beschreibung der erfindungsgemäßen Stoffe der Formel (I) für diesen Substituenten als bevorzugt genannt wurden.

Die Hydroxylamine der Formel (VI) sind allgemein bekannte Verbindungen der organischen Chemie.

Die bei der Durchführung des erfindungsgemäßen Verfahrens (d) als Ausgangsstoffe zu verwendenden Verbindungen der Formel (Ib) sind erfindungsgemäße Stoffe.

Die außerdem für das erfindungsgemäße Verfahren (d) als Ausgangsstoffe zu verwendenden Halogenide sind durch die Formel (VII) allgemein definiert. In dieser Formel steht $Y^4$ bevorzugt für die Bedeutungen, die bereits im Zusammenhang mit der Beschreibung der erfindungsgemäßen Stoffe der Formel (I) für $Y^2$, außer Wasserstoff, als bevorzugt genannt wurden.

Die Halogenide der Formel (VII) sind allgemein bekannte Verbindungen der organischen Chemie.

Als Verdünnungsmittel kommen für die erfindungsgemäßen Verfahren (a) und (b) unter den Reaktionsbedingungen inerte organische Lösungsmittel infrage. Hierzu gehören vorzugsweise Alkohole, wie z. B. Ethanol, Methoxyethanol oder Propanol ; Ketone, wie z. B. 2-Butanol ; Nitrile, wie z. B. Acetonitril ; Ester, wie z. B. Essigester ; Ether, wie z. B. Dioxan ; aromatische Kohlenwasserstoffe, wie z. B. Benzol und Toluol ; oder Amide, wie z. B. Dimethylformamid.

Als Basen kommen für die erfindungsgemäßen Umsetzungen alle üblicherweise verwendbaren anorganischen und organischen Basen in Betracht. Hierzu gehören vorzugsweise Alkalicarbonate, wie z. B. Natrium- und Kaliumcarbonat ; Alkalihydroxide, wie z. B. Natriumhydroxid ; Alkalialkoholate, wie z. B. Natrium- und Kalium-methylat und -ethylat ; Alkalihydride, wie z. B. Natriumhydrid ; sowie niedere tertiäre Alkylamine, Cycloalkylamine und Aralkylamine, wie insbesondere Triethylamin.

Die Reaktionstemperaturen können bei der Durchführung der erfindungsgemäßen Verfahren (a) und (b) in einem größeren Bereich variiert werden. Im allgemeinen arbeitet man bei Temperaturen zwischen 60 °C und 150 °C.

Bei der Durchführung des erfindungsgemäßen Verfahrens (a) setzt man vorzugsweise auf 1 Mol Oxiran der Formel (II) 1 bis 2 Mol Azol und gegebenenfalls katalytische bis 2-molare Mengen an Base ein ; bei der Durchführung des erfindungsgemäßen Verfahrens (b) setzt man vorzugsweise auf 1 Mol Triazolylmethyl-oxiran der Formel (IV) 1 bis 2 Mol (Thio)Phenol der Formel (V) und gegebenenfalls katalytische bis 2-molare Mengen Base ein. Die Isolierung der Endprodukte erfolgt jeweils in allgemein üblicher Weise.

Als Verdünnungsmittel kommen für das erfindungsgemäße Verfahren (c) vorzugsweise Alkohole und Wasser, bzw. Gemische beider infrage.

Die Reaktionstemperaturen können beim Verfahren (c) in einem größeren Bereich variiert werden. Im allgemeinen arbeitet man bei Temperaturen zwischen 20 °C und 120 °C, vorzugsweise zwischen 50 °C und 100 °C.

Bei der Durchführung des erfindungsgemäßen Verfahrens (c) setzt man auf 1 Mol der Verbindung der Formel (Ia) vorzugsweise 1 bis 1,5 Mol Hydroxylamin-Derivat der Formel (VI) ein. Die Isolierung der Verbindungen der Formel (I) erfolgt nach üblichen Methoden.

Nach einer bevorzugten Ausführungsform des Verfahrens (c) werden die Hydroxylamin-Derivate der

Formel (VI) in Form ihrer Salze, insbesondere als Hydrochloride, gegebenenfalls in Gegenwart eines Säurebindemittels, wie beispielsweise Natriumacetat, eingesetzt (vgl. auch die Herstellungsbeispiele).

Für die erfindungsgemäße Umsetzung gemäß Verfahren (d) kommen als Verdünnungsmittel inerte organische Lösungsmittel infrage. Hierzu gehören vorzugsweise Ether, wie Tetrahydrofuran und Dioxan ; aromatische Kohlenwasserstoffe, wie Toluol und Benzol ; in einzelnen Fällen auch chlorierte Kohlenwasserstoffe, wie Chloroform, Methylenchlorid oder Tetrachlorkohlenstoff ; sowie Hexamethylphosphorsäure-triamid, Säureamide, wie Dimethylformamid und Sulfoxide, wie Dimethylsulfoxid.

Die erfindungsgemäße Umsetzung gemäß Verfahren (d) wird gegebenenfalls in Gegenwart einer starken Base durchgeführt. Hierzu gehören vorzugsweise Alkalimetallamide, -hydride, -hydroxide und -carbonate, wie beispielsweise Natriumamid, -carbonat, -hydroxid oder -hydrid und Kaliumamid, -carbonat, -hydroxid oder -hydrid, sowie quarternäre Ammoniumhydroxide und Phosphoniumhydroxide, wie beispielsweise Tetramethylammoniumhydroxid, Benzyltrimethyl-ammoniumhydroxid oder Dibenzyldimethyl-ammoniumhydroxid und Tetraphenylphosphoniumhydroxid oder Methyltriphenyl-phosphoniumhydroxid.

Die Reaktionstemperaturen können beim Verfahren (d) in einem größeren Bereich varriert werden. Im allgemeinen arbeitet man zwischen 20 und 150 °C, vorzugsweise bei Raumtemperatur. In einzelnen Fällen ist es vorteilhaft bei der Siedetemperatur des Lösungsmittels, beispielsweise zwischen 60 und 100 °C, zu arbeiten.

Bei der Durchführung des erfindungsgemäßen Verfahrens (d) setzt man auf 1 Mol der Verbindungen der Formel (Ib) vorzugsweise 1 bis 3 Mol Halogenid der Formel (VII) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform des Verfahrens (d) wird die erfindungsgemäße Umsetzung in einem Zweiphasensystem, wie beispielsweise wäßrige Natron- oder Kalilauge/Toluol oder Methylenchlorid, unter Zusatz von 0,01 bis 1 Mol eines Phasen-Transfer-Katalysators, wie beispielsweise Ammonium- oder Phosphoniumverbindungen, durchgeführt, wobei in der organischen Phase oder an den Grenzflächen die Alkoholate entstehen und mit den in der organischen Phase befindlichen Halogeniden umgesetzt werden.

Die nach den erfindungsgemäßen Verfahren erhältlichen Verbindungen der Formel (I) können in Säureadditions-Salze bzw. Metallsalz-Komplexe überführt werden.

Zur Herstellung von physiologisch verträglichen Säureadditionssalzen der Verbindungen der Formel (I) kommen vorzugsweise folgende Säuren infrage : Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure.

Die Säureadditionssalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten : Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie gegen Gerstenmehltau (Erysiphe graminis) und die Streifenkrankheit der Gerste (Drechslera graminea), sowie gegen Cochliobolus sativus eingesetzt werden, ferner zur Bekämpfung von Podosphaera-Arten, wie z. B. gegen den Erreger des Apfelmehltaus (Podosphaera

leucotricha), von Botrytis-Arten, wie z. B. gegen den Erreger des Grauschimmels (Botrytis cinerea), sowie auch zur Bekämpfung von Reiskrankheiten, wie Pyricularia oryzae und Pellicularia sasakii. Außerdem besitzen die erfindungsgemäßen Verbindungen eine gute Wirkung gegen Puccinia und Pyrenophora teres an Getreide.

Hervorzuheben ist, daß die erfindungsgemäßen Stoffe nicht nur eine protektive Wirkung haben, sondern teilweise auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsions-konzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen infrage : Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen infrage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen infrage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln ; als Emulgier- und/oder schaumerzeugende Mittel kommen infrage : z. B. nichtionogene und anionische Emulgatoren, wie Polyoxyethylen-Fettsäureester, Polyoxyethylen-Fettalkohol-ether, z. B. Alkylarylpolyglykol-ether, Alkylsulfonate, Alkylsulfate, Arylsulfonate sowie Eiweißhydrolysate ; als Dispergiermittel kommen infrage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen, bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 Gew.-%, am Wirkungsort erforderlich.

## Herstellungsbeispiele

Beispiel 1

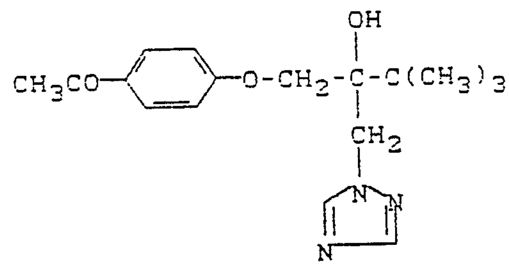

(Verfahren b)

Eine Lösung von 28,96 g (0,16 Mol) 2-tert.-Butyl-2-(1,2,4-triazol-1-yl-methyl)-oxiran in 50 ml n-Propanol wird unter Rühren bei Raumtemperatur zu einer Lösung von 24,5 g (0,18 Mol) p-Hydroxyacetophenon und 0,46 g (0,02 Mol) Natrium in 150 ml n-Propanol getropft. Das Reaktionsgemisch wird anschließend 4 Tage unter Rückfluß erhitzt. Danach wird eingeengt, der Rückstand in Essigester aufgenommen und zweimal mit 1n Natronlauge, zweimal mit Wasser sowie einmal mit gesättigter Kochsalzlösung gewaschen. Die Essigesterphase wird über Natriumsulfat getrocknet und eingeengt. Der Rückstand wird über eine Kieselgelsäule chromatographiert (Dichlormethan/Essigester = 4 : 1). Das erhaltene Produkt kristallisiert nach Zugabe von Ether. Man erhält 26,9 g (53 % der Theorie) 2-(4-Acetophenoxymethyl)-3,3-dimethyl-1-(1,2,4-triazol-1-yl)-2-butanol vom Schmelzpunkt 98-99 °C.

Beispiel 2

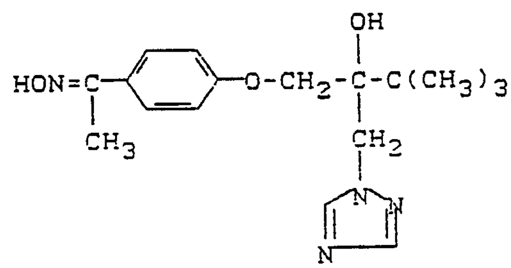

(Verfahren c)

43,5 g (0,137 Mol) 2-(4-Acetophenoxymethyl)-3,3-dimethyl-1-(1,2,4-triazol-1-yl)-2-butanol (Beispiel 1), 14,28 g (0,205 5 Mol) Hydroxylamin-hydrochlorid und 16,85 g (0,205 5 Mol) Natriumacetat werden in einem Gemisch von 150 ml Wasser und 250 ml Ethanol gelöst und über Nacht unter Rückfluß erhitzt. Danach wird das Reaktionsgemisch eingeengt, der Rückstand in Wasser/Essigester aufgenommen und mit 1n Natronlauge basisch gestellt. Die organische Phase wird abgetrennt und die wäßrige Phase zweimal mit Essigester extrahiert. Die vereinigten organischen Phasen werden einmal mit verdünnter und zweimal mit gesättigter Kochsalzlösung gewaschen, über Natriumsulfat getrocknet und eingeengt. Nach Umkristallisation des Rückstandes aus Acetonitril erhält man 34,8 g (76,5 % der Theorie) 3,3-Dimethyl-2-[4-(1-hydroxyiminoethyl)-phenoxymethyl]-1-(1,2,4-triazol-1-yl)-2-butanol vom Schmelzpunkt 129-132 °C.

Beispiel 3

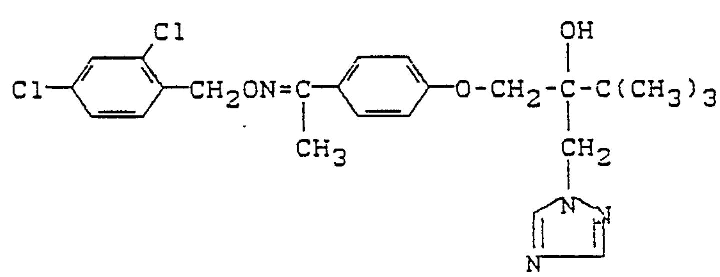

(Verfahren d)

1,44 g (0.06 Mol) Natriumhydrid (80 %ig in Paraffinöl) werden zu einer Lösung von 14,94 g (0,045 Mol) 3,3-Dimethyl-2-[4-(1-hydroximinoethyl)-phenoxymethyl]-1-(1,2,4-triazol-1-yl)-2-butanol (Beispiel 2) in 120 ml absolutem Dimethylsulfoxid gegeben. Man läßt das Reaktionsgemisch bei Raumtemperatur ca. 1

Stunde nachrühren, bis eine klare Lösung entstanden ist. Anschließend wird mit 8,33 ml (0,06 Mol) 2,4-Dichlorbenzylchlorid versetzt und das Reaktionsgemisch 1,5 Stunden bei Raumtemperatur nachgerührt. Danach versetzt man vorsichtig mit Wasser und Eisessig und engt ein. Der Rückstand wird in Essigester aufgenommen, mehrmals mit verdünnter Kochsalzlösung gewaschen, über Natriumsulfat getrocknet und eingeengt. Der Rückstand wird über eine Kieselgelsäule chromatographiert (Dichlormethan/Essigester = 4 : 1) und aus Acetonitril umkristallisiert. Man erhält 16,2 g (73 % der Theorie) 2-{4-[1-(2,4-Dichlorbenzyloximino)-ethyl]-phenoxymethyl}-3,3-dimethyl-1-(1,2,4-triazol-1-yl)-2-butanol vom Schmelzpunkt 101 °C.

Beispiel 4

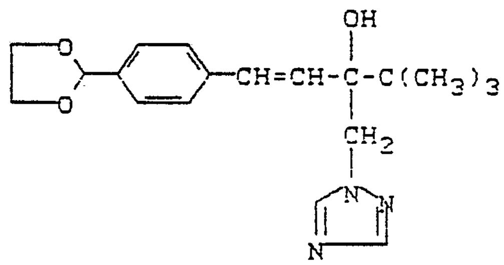

(Verfahren a)

25,6 g (0,093 Mol) 2-{2-[4-(1,3-Dioxolan-2-yl)-phenyl]-ethenyl}-2-tert.-butyl-oxiran in 47 ml absolutem Dimethylformamid werden bei Raumtemperatur zu einer Lösung von Natriumtriazolid — hergestellt durch langsames Eintragen von 12,9 g (0,187 Mol) 1,2,4-Triazol in eine Suspension von 5,6 g (0,187 Mol) Natriumhydrid (80 %ig in Paraffinöl) in 187 ml absolutem Dimethylformamid getropft. Das Reaktionsgemisch wird 4 Stunden bei 80 °C gerührt und über Nacht bei Raumtemperatur stehengelassen. Danach wird das Reaktionsgemisch auf Eis/Wasser gegossen und mehrmals mit Essigester extrahiert. Die vereinigten Essigesterphasen werden zweimal mit Wasser und einmal mit gesättigter Kochsalzlösung gewaschen, über Natriumsulfat getrocknet und eingeengt. Der Rückstand wird über eine Kieselgelsäule chromatographiert (Dichlormethan/Essigester = 2 : 1) und aus n-Hexan/Essigester umkristallisiert. Man erhält 7,75 g (24 % der Theorie) 3,3-Dimethyl-2-{2-[4-(1,3-Dioxolan-2-yl)-phenyl]-ethenyl}-1-(1,2,4-triazol-1-yl)-2-butanol vom Schmelzpunkt 109,5-110 °C.

Herstellung des Ausgangsproduktes

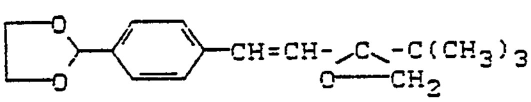

8,1 g (0,13 Mol) Iodmethan werden langsam zu einer Lösung von 9,9 ml (0,135 Mol) Dimethylsulfid in 75 ml absolutem Dimethylsulfoxid und 35 ml absolutem Tetrahydrofuran getropft, wobei die Innentemperatur 35 °C nicht übersteigen darf. Diese Suspension wird über Nacht bei Raumtemperatur nachgerührt und anschließend portionsweise mit 15,7 g (0,14 Mol) Kalium-tert.-Butylat versetzt. Es wird 30 Minuten bei Raumtemperatur nachgerührt, auf 0 °C abgekühlt und portionsweise mit 26 g (0,1 Mol) 4,4-Dimethyl-1-[4-(1,3-dioxolan-2-yl)-phenyl]-1-penten-3-on versetzt. Das Reaktionsgemisch wird über Nacht bei Raumtemperatur nachgerührt, anschließend mit Wasser versetzt und dreimal mit Toluol extrahiert. Die vereinigten Toluolphasen werden mit Wasser und gesättigter Kochsalzlösung gewaschen, über Natriumsulfat getrocknet und eingeengt. Man erhält 25,6 g rohes 2-{2-[4-(1,3-Dioxolan-2-yl)-phenyl]-ethenyl}-2-tert.-butyl-oxiran als Öl, das direkt weiter umgesetzt wird.

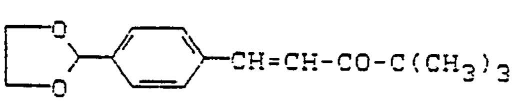

100 g (0,561 2 Mol) 4-(1,3-Dioxolan-2-yl)-benzaldehyd und 56,1 g (0,56 Mol) Pinakolin werden in einem Gemisch von 280 ml Ethanol und 56 ml Wasser gelöst und bei Raumtemperatur mit 17 ml 10 %iger Natronlauge versetzt. Man läßt 1 Stunde nachrühren, versetzt mit 0,56 g festem Natriumhydroxid und läßt 2 Tage nachrühren. Danach wird der Niederschlag abgesaugt, mit 200 ml Ethanol/Wasser (1 : 1) und danach mit Wasser gewaschen, getrocknet und zweimal aus Ethanol umkristallisiert. Man erhält 56,5 g (39 % der Theorie) 4,4-Dimethyl-1-[4-(1,3-dioxolan-2-yl)-phenyl]-1-penten-3-on vom Schmelzpunkt 79-81 °C.

Beispiel 5

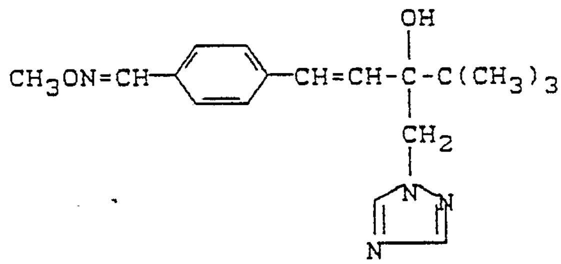

(Verfahren c)

1 g (0,002 9 Mol) 3,3-Dimethyl-2-{2-[4-(1,3-dioxolan-2-yl)-phenyl]-ethenyl}-1-(1,2,4-triazol-1-yl)-2-butanol (Beispiel 4), 0,42 g (0,005 Mol) O-Methyl-hydroxylamin-hydrochlorid und 0,41 g (0,005 Mol) Natriumacetat werden in 20 ml Ethanol und 15 ml Wasser gelöst und 2 Stunden unter Rückfluß erhitzt. Das Reaktionsgemisch wird eingeengt, der Rückstand in Essigester aufgenommen, zweimal mit Wasser, einmal mit gesättigter Kochsalzlösung gewaschen, über Natriumsulfat getrocknet und eingeengt. Der Kristalline Rückstand wird mit wenig Ether gewaschen und aus n-Hexan/-Essigester umkristallisiert. Man erhält 0,67 g (70 % der Theorie) 3,3-Dimethyl-2-[4-methoximinomethylphenyl)-ethenyl]-1-(1,24-triazol-1-yl)-2-butanol vom Schmelzpunkt 140-142 °C.

In analoger Weise und entsprechend den erfindungsgemäßen Verfahren (a) bis (d) werden die nachfolgenden Verbindungen der allgemeinen Formel

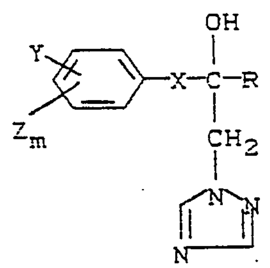

(I)

erhalten :

| Bsp. Nr. | Y | $Z_m$ | X | R | Schmelzpunkt (°C) bzw. $n_D^{20}$ |
|---|---|---|---|---|---|
| 6 | $4-CH_3ON=CH-$ | - | $-OCH_2-$ | $-C(CH_3)_3$ | 92-93 |
| 7 | $4-CH_3ON=C(CH_3)-$ | - | $-OCH_2-$ | $-C(CH_3)_3$ | 92-94 |
| 8 | $4-CH_2=CH-CH_2ON=C(CH_3)-$ | - | $-OCH_2-$ | $-C(CH_3)_3$ | 1,5445 |
| 9 | $4-CH_3CO-$ | - | $-OCH_2-$ | $-C(CH_3)_2CH_2F$ | 111 |
| 10 | $4-OHC-$ | - | $-OCH_2-$ | $-C(CH_3)_3$ | 1,5491 |
| 11 | $4-CH_3CO-$ | - | $-OCH_2-$ | $-C(CH_3)_2$<br>$CH_2OCH_3$ | 80-81 |
| 12 | $4-OHC-$ | - | $-OCH_2-$ | $-C(CH_3)_2$<br>$CH_2OCH_3$ | 1,5457 |
| 13 | $4-CH_3ON=C(CH_3)-$ | - | $-OCH_2-$ | $-C(CH_3)_2CH_2F$ | 94,5-95 |

(Fortsetzung)

| Bsp. Nr. | Y | $Z_m$ | X | R | Schmelzpunkt (°C) bzw. $n_D^{20}$ |
|---|---|---|---|---|---|
| 14 | $4-CH_3ON=CH-$ | - | $-CH_2CH_2-$ | $-C(CH_3)_3$ | 77-79 |
| 15 | $4-CH_3ON=CH-$ | - | $-OCH_2-$ | $-C(CH_3)_2$<br>$CH_2OCH_3$ | 1,5430 |
| 16 | $4-CH_3ON=C(CH_3)-$ | - | $-OCH_2-$ | $-C(CH_3)_2$<br>$CH_2OCH_3$ | 62-65 |
| 17 | $4-CH_2=CH-CH_2-ON=CH-$ | - | $-OCH_2-$ | $-C(CH_3)_3$ | 1,5560 |
| 18 | 4 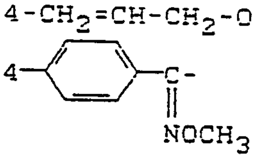 | - | $-OCH_2-$ | $-C(CH_3)_3$ | 1,5692 |
| 19 | 3 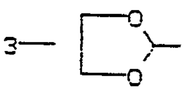 | - | $-OCH_2-$ | $-C(CH_3)_3$ | 73-75 |
| 20 | 4 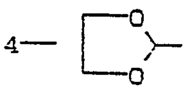 | - | - | 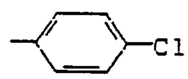 | 135-36,5 |
| 21 | $3-CH_3ON=CH-$ | - | $-OCH_2-$ | $-C(CH_3)_3$ | 76-78 |
| 22 | $2-CH_3CO-$ | - | $-OCH_2-$ | $-C(CH_3)_3$ | 105,5-07 |
| 23 | $2-CH_3ON=C(CH_3)-$ | - | $-OCH_2-$ | $-C(CH_3)_3$ | 71-73 |

## Verwendungsbeispiele

In den nachfolgenden Verwendungsbeispielen werden die nachstehend angegebenen Substanzen als Vergleichsverbindungen eingesetzt :

(A) 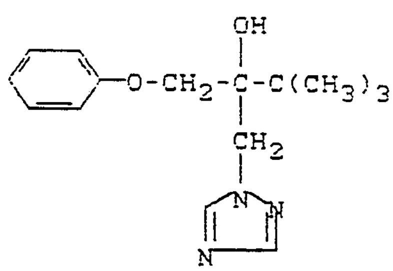

(B) 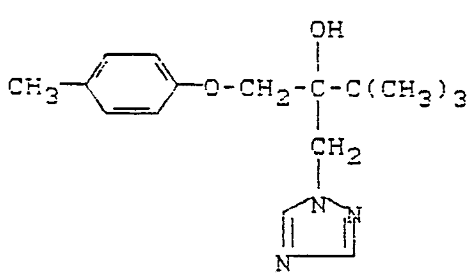

(C) 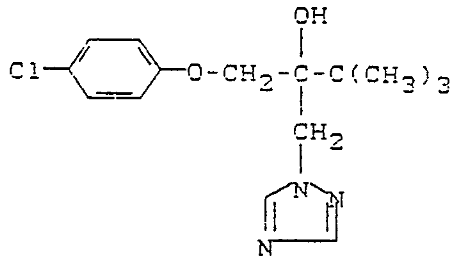

(D) 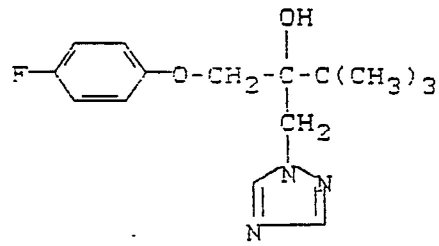

Beispiel A

Cochliobolus sativus-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 °C rel. Luftfeuchtigkeit in einer Inkubationskabine.

Die Pflanzen werden in einem Gewächshaus bei einer Temperatur von 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber den Stand der Technik zeigen bei diesem Test z. B. die Verbindungen gemäß folgender Herstellungsbeispiele : 6 und 7.

Beispiel B

Erysiphe-Test (Gerste)/Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 × 12 Korn 2 cm tief in eine Standarderde. 7 Tage nach der Aussaat, wenn die jungen Pflanzen ihr erstes Blatt entfaltet haben, werden sie mit Sporen von Erysiphe graminis f.sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt in diesem Test z. B. die Verbindung gemäß folgendem Herstellungsbeispiel : 7.

Beispiel C

Drechslera graminea-Test (Gerste)/Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4 °C aus. Dabei wird die Keimung der Gerste und gegebenenfalls auch der Pilzsporen eingeleitet. Anschließend sät man die vorgekeimte Gerste mit 2 × 50 Korn 3 cm tief in eine Standarderde und kultiviert sie im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen in diesem Test z. B. die Verbindungen gemäß folgender Herstellungsbeispiele : 6 und 7.

Beispiel D

Podosphaera-Test (Apfel)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23 °C und einer relativen Luftfeuchtigkeit von ca. 70 % aufgestellt.

9 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 6 und 7.

Beispiel E

Botrytis-Test (Bohne)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigt in diesem Test z. B. die Verbindung gemäß folgendem Herstellungsbeispiel : 6.

Beispiel F

Pyricularia-Test (Reis)/protektiv

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine gute Wirksamkeit zeigt bei diesem Test z. B. die Verbindung gemäß folgendem Herstellungsbeispiel : 6.

Beispiel G

Pyricularia-Test (Reis) systemisch

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegeben Menge Emulgator auf die gewünschte Konzentration.

Zur Prüfung aus systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Eine gute Wirksamkeit zeigt bei diesem Test z. B. die Verbindung gemäß folgendem Herstellungsbeispiel : 7.

Beispiel H

Pellicularia-Test (Reis)

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25 °C und 100 % relativer Luftfeuchtigkeit aufgestellt.

5 bis 8 Tage nach der Inokulation erfolgt die Auswertung des Krankheitsbefalles.

Eine gute Wirksamkeit zeigt bei diesem Test z. B. die Verbindung gemäß folgendem Herstellungsbeispiel : 6.

**Patentansprüche**

1. Substituierte 1-Hydroxyethyl-triazolyl-Derivate der allgemeinen Formel

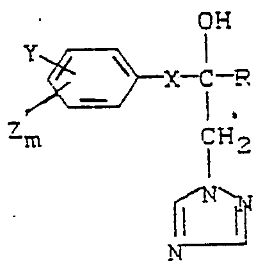

(I)

in welcher

R für geradkettiges oder verzweigtes Alkyl mit 1 bis 4 Kohlenstoffatomen, für gegebenenfalls einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen und Alkoxy mit 1 bis 2 Kohlenstoffatomen, ferner für gegebenenfalls einfach oder mehrfach, gleich oder verschieden substituiertes Phenyl steht, wobei als Substituenten die bei $R^3$ genannten Phenylsubstituenten infrage kommen, sowie für die Gruppierungen

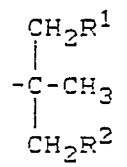

und

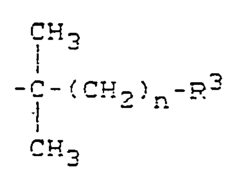

steht ;

$R^1$ für Wasserstoff oder Halogen steht ;

$R^2$ für Halogen steht ;

$R^3$ für Alkyl, Alkoxy und Alkylthio mit jeweils 1 bis 4 Kohlenstoffatomen, für Halogenalkoxy und Halogenalkylthio mit jeweils 1 bis 2 Kohlenstoffatomen und 1 bis 5 gleichen oder verschiedenen Halogenatomen steht, ferner für Alkenyl mit 2 bis 6 Kohlenstoffatomen, für Alkoxycarbonyl mit 1 bis 4 Kohlenstoffatomen im Alkylteil, für Cyano sowie für jeweils gegebenenfalls einfach oder mehrfach substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil, steht, wobei jeweils als Phenylsubstituenten genannt seien : Halogen, Alkyl mit 1 bis 4 Kohlenstoffatomen ; Alkoxy und Alkylthio mit jeweils 1 bis 2 Kohlenstoffatomen ; Halogenalkyl, Halogenalkoxy und Halogenalkylthio mit jeweils 1 bis 2 Kohlenstoff- und 1 bis 5 gleichen oder verschiedenen Halogenatomen, Cyclohexyl, Dialkylamino mit 1 bis 4 Kohlenstoffatomen in jedem Alkylteil, Nitro, Cyano, Alkoxycarbonyl mit 1 bis 4 Kohlenstoffatomen im Alkylteil, sowie gegebenenfalls durch Halogen substituiertes Phenyl,

n für die Zahlen 0, 1 oder 2 steht ;

X für die Gruppierungen $-OCH_2-$, $-SCH_2-$, $-(CH_2)_p-$ oder $-CH=CH-$ steht ;

Y für die Gruppierungen $-CO-Y^1$, $-C(OR^4)_2-Y^1$ und

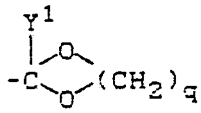

sowie auch für die Gruppierung $-C(Y^1)=N-OY^2$ steht ;

$Y^1$ für Wasserstoff, für Alkyl mit 1 bis 4 Kohlenstoffatomen, für Alkenyl und Alkinyl mit jeweils 2 bis 4 Kohlenstoffatomen, für gegebenenfalls einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen und Alkoxy mit 1 bis 2 Kohlenstoffatomen, sowie für jeweils gegebenenfalls einfach oder mehrfach, gleich oder verschieden substituiertes Phenyl oder Benzyl steht, wobei als Substituenten die bei $R^3$ genannten Phenylsubstituenten infrage kommen ;

$Y^2$ für Wasserstoff, für geradkettiges oder verzweigtes Alkyl mit 1 bis 4 Kohlenstoffatomen, für Alkenyl und Alkinyl mit jeweils 2 bis 6 Kohlenstoffatomen, für gegebenenfalls einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen und Alkoxy mit 1 bis 2 Kohlenstoffatomen ; sowie für gegebenenfalls einfach oder mehrfach, gleich oder verschieden substituiertes Benzyl steht, wobei als Substituenten die bei $R^3$ genannten Phenylsubstituenten infrage kommen ;

$R^4$ für Alkyl mit 1 bis 4 Kohlenstoffatomen steht ;

q für die Zahlen 2 oder 3 steht ;

Z für Halogen, für Alkyl, Alkoxy und Alkylthio mit jeweils 1 bis 4 Kohlenstoffatomen, sowie für Halogenalkyl, Halogenalkoxy oder Halogenalkylthio mit jeweils 1 bis 2 Kohlenstoffatomen und 1 bis 5 gleichen oder verschiedenen Halogenatomen steht ;

m für die Zahlen 0, 1 oder 2 steht und

p für die Zahlen 0, 1 oder 2 steht ;

sowie deren Säureadditions-Salze und Metallsalz-Komplexe.

2. Verbindungen der allgemeinen Formel (I) in Anspruch 1, wobei

R für tert.-Butyl oder Isopropyl steht, für jeweils gegebenenfalls einfach bis dreifach, gleich oder verschieden substituiertes Cyclopropyl, Cyclopentyl oder Cyclohexyl steht, wobei als Substituenten genannt seien : Methyl, Ethyl, Isopropyl, Methoxy und Ethoxy, ferner für gegebenenfalls einfach bis dreifach, gleich oder verschieden durch Fluor, Chlor, Methyl, Trifluormethyl, Phenyl und Chlorphenyl substituiertes Phenyl steht, sowie für die Gruppierungen

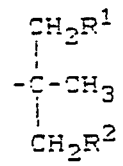 und 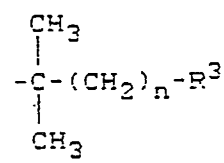

steht, wobei

$R^1$ für Wasserstoff, Fluor oder Chlor steht ;

$R^2$ für Fluor oder Chlor steht ;

$R^3$ für Methyl, Ethyl, Propyl, Methoxy, Ethoxy, Methylthio, Ethylthio, Trifluormethoxy, Trifluormethylthio, Vinyl, Methoxycarbonyl, Ethoxycarbonyl, Cyano sowie für jeweils gegebenenfalls einfach bis

zweifach, gleich oder verschieden substituiertes Phenyl, Phenoxy, Phenylthio, Phenylmethoxy oder Phenylmethylthio steht, wobei die fünf letztgenannten Reste substituiert sein können durch Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl ; und

n für die Zahlen 0,1 oder 2 steht ;

X für die Gruppierungen $—OCH_2—$, $—SCH_2—$, $—(CH_2)_p—$, oder $—CH = CH—$ steht ;

Y für die Gruppierungen $—CO—Y^1$, $—C(OR^4)_2—Y^1$ und

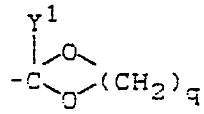

sowie auch für die Gruppierung $—C(Y^1) = N—OY^2$ steht ;

$Y^1$ für Wasserstoff, Methyl, Ethyl, Isopropyl oder für gegebenenfalls einfach bis zweifach, gleich oder verschieden durch Fluor, Chlor, Methyl oder Trifluormethyl substituiertes Phenyl steht ;

$Y^2$ für Wasserstoff, Methyl, Ethyl, n-Propyl, n-Butyl, Allyl, Propargyl oder für gegebenenfalls einfach bis zweifach, gleich oder verschieden durch Fluor, Chlor, Methyl, Trifluormethyl oder Trifluormethoxy substituiertes Benzyl steht ;

$R^4$ für Methyl, Ethyl oder Propyl steht ; und

q für die Zahlen 2 oder 3 steht ;

Z für Fluor, Chlor, Brom, Methyl, Methoxy, Methylthio, Trifluormethyl, Trifluormethoxy oder Trifluormethylthio steht, und

m für die Zahlen 0, 1 oder 2 steht ; und

p für die Zahlen 0, 1 oder 2 steht.

3. Verfahren zur Herstellung von substituierten 1-Hydroxyethyl-triazolyl-Derivaten der allgemeinen Formel

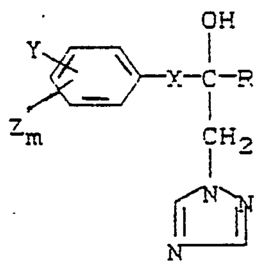

(I)

in welcher

R für geradkettiges oder verzweigtes Alkyl mit 1 bis 4 Kohlenstoffatomen, für gegebenenfalls einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen und Alkoxy mit 1 bis 2 Kohlenstoffatomen, ferner für gegebenenfalls einfach oder mehrfach, gleich oder verschieden substituiertes Phenyl steht, wobei als Substituenten die bei $R^3$ genannten Phenylsubstituenten infrage kommen, sowie für die Gruppierungen

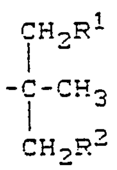

und

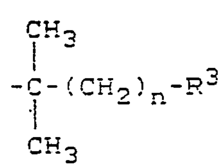

steht ;

$R^1$ für Wasserstoff oder Halogen steht ;

$R^2$ für Halogen steht ;

$R^3$ für Alkyl, Alkoxy und Alkylthio mit jeweils 1 bis 4 Kohlenstoffatomen, für Halogenalkoxy und Halogenalkylthio mit jeweils 1 bis 2 Kohlenstoffatomen und 1 bis 5 gleichen oder verschiedenen Halogenatomen steht, ferner für Alkenyl mit 2 bis 6 Kohlenstoffatomen, für Alkoxycarbonyl mit 1 bis 4 Kohlenstoffatomen im Alkylteil, für Cyano sowie für jeweils gegebenenfalls einfach oder mehrfach substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil, steht, wobei jeweils als Phenylsubstituenten genannt seien : Halogen, Alkyl mit 1 bis 4 Kohlenstoffatomen ; Alkoxy und Alkylthio mit jeweils 1 bis 2 Kohlenstoffatomen ; Halogenalkyl, Halogenalkoxy und Halogenalkylthio mit jeweils 1 bis 2 Kohlenstoff- und 1 bis 5 gleichen oder verschiedenen Halogenatomen, Cyclohexyl, Dialkylamino mit 1 bis 4

Kohlenstoffatomen in jedem Alkylteil, Nitro, Cyano, Alkoxycarbonyl mit 1 bis 4 Kohlenstoffatomen im Alkylteil, sowie gegebenenfalls durch Halogen substituiertes Phenyl,

n für die Zahlen 0, 1 oder 2 steht ;

X für die Gruppierungen $—OCH_2—$, $—SCH_2—$, $—(CH_2)_p—$ oder $—CH = CH—$ steht ;

Y für die Gruppierungen $—CO—Y^1$, $—C(OR^4)_2—Y^1$ und

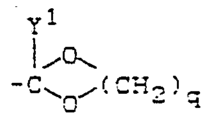

sowie auch für die Gruppierung $—C(Y^1) = N—OY^2$ steht ;

$Y^1$ für Wasserstoff, für Alkyl mit 1 bis 4 Kohlenstoffatomen, für Alkenyl und Alkinyl mit jeweils 2 bis 4 Kohlenstoffatomen, für gegebenenfalls einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen und Alkoxy mit 1 bis 2 Kohlenstoffatomen, sowie für jeweils gegebenenfalls einfach oder mehrfach, gleich oder verschieden substituiertes Phenyl oder Benzyl steht, wobei als Substituenten die bei $R^3$ genannten Phenylsubstituenten infrage kommen ;

$Y^2$ für Wasserstoff, für geradkettiges oder verzweigtes Alkyl mit 1 bis 4 Kohlenstoffatomen, für Alkenyl und Alkinyl mit jeweils 2 bis 6 Kohlenstoffatomen, für gegebenenfalls einfach oder mehrfach, gleich oder verschieden substituiertes Cycloalkyl mit 3 bis 7 Kohlenstoffatomen steht, wobei als Substituenten genannt seien : Halogen, Alkyl mit 1 bis 4 Kohlenstoffatomen und Alkoxy mit 1 bis 2 Kohlenstoffatomen ; sowie für für gegebenenfalls einfach oder mehrfach, gleich oder verschieden substituiertes Benzyl steht, wobei als Substituenten die bei $R^3$ genannten Phenylsubstituenten infrage kommen ;

$R^4$ für Alkyl mit 1 bis 4 Kohlenstoffatomen steht ;

q für die Zahlen 2 oder 3 steht ;

Z für Halogen, für Alkyl, Alkoxy und Alkylthio mit jeweils 1 bis 4 Kohlenstoffatomen, sowie für Halogenalkyl, Halogenalkoxy oder Halogenalkylthio mit jeweils 1 bis 2 Kohlenstoffatomen und 1 bis 5 gleichen oder verschiedenen Halogenatomen steht ;

m für die Zahlen 0, 1 oder 2 steht und

p für die Zahlen 0, 1 oder 2 steht ;

sowie deren Säureadditions-Salzen und Metallsalz-Komplexen, dadurch gekennzeichnet, daß man

a) Oxirane der Formel

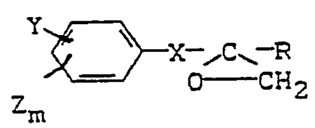

(II)

in welcher R, X, Y, Z und m die oben angegebene Bedeutung haben, mit 1,2,4-Triazol der Formel

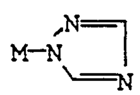

(III)

in welcher M für Wasserstoff oder ein Alkalimetall steht, in Gegenwart eines Verdünnungsmittels und gegebenenfalls in Gegenwart einer Base umsetzt, oder

b) Triazolylmethyl-oxirane der Formel

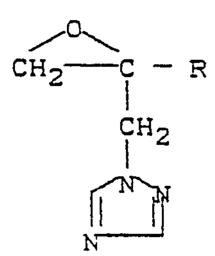

(IV)

in welcher R die oben angegebene Bedeutung hat, mit (Thio)Phenolen der Formel

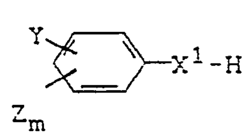

(V)

in welcher
Y, Z und m die oben angegebene Bedeutung haben und
$X^1$ für Sauerstoff oder Schwefel steht,
in Gegenwart eines Verdünnungsmittels und gegebenenfalls in Gegenwart einer Base umsetzt, oder
c) die nach den Verfahren (a) und (b) erhältlichen erfindungsgemäßen Verbindungen der Formel

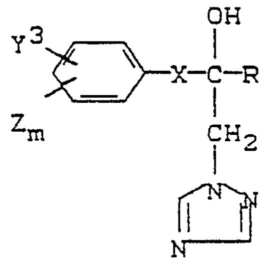
(Ia)

in welcher
R, X, Z und m die oben angegebene Bedeutung haben und
$Y^3$ für die Gruppierungen $—CO—Y^1$, $—C(OR^4)_2Y^1$ und

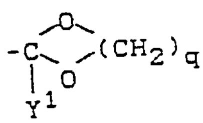

steht, mit Hydroxylamin-Derivaten der Formel

$$H_2N—O—Y^2 \qquad (VI)$$

in welcher $Y^2$ die oben angegebene Bedeutung hat, in Gegenwart eines Verdünnungsmittels umsetzt, oder
d) die nach den Verfahren a), b) und c) erhältlichen erfindungsgemäßen Verbindungen der Formel

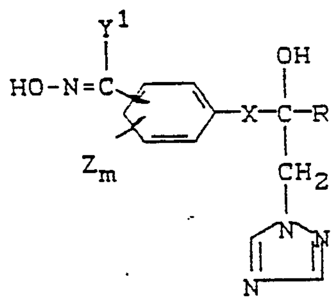
(Ib)

in welcher R, X, $Y^1$, Z und m die oben angegebene Bedeutung haben, mit Halogeniden der Formel

$$Y^4—Hal \qquad (VII)$$

in welcher
Hal für Chlor, Brom oder Iod steht und
$Y^4$ für die Bedeutungen von $Y^2$, außer für Wasserstoff, steht,
in Gegenwart eines Verdünnungsmittels und gegebenenfalls in Gegenwart einer Base umsetzt, und gegebenenfalls an die nach obigen Verfahren erhaltenen Verbindungen der Formel (I) anschließend eine Säure oder ein Metallsalz addiert.

4. Pflanzenschutzmittel, gekennzeichnet durch einen Gehalt an mindestens einem substituierten 1-Hydroxyethyl-triazolyl-Derivat der Formel (I) in Anspruch 1 bzw. an einem Säureadditions-Salz oder Metallsalz-Komplex einer Verbindung der Formel (I).

5. Fungizide Mittel, gekennzeichnet durch einen Gehalt an mindestens einem substituierten 1-Hydroxyethyltriazolyl-Derivat der Formel (I) in Anspruch 1 bzw. an einem Säureadditions-Salz oder Metallsalz-Komplex einer Verbindung der Formel (I).

6. Verfahren zur Bekämpfung von Pilzen, dadurch gekennzeichnet, daß man substituierte 1-Hydroxyethyltriazolyl-Derivate der Formel (I) in Anspruch 1 bzw. deren Säureadditions-Salze oder Metallsalz-Komplexe auf Pilze oder ihren Lebensraum einwirken läßt.

7. Verwendung von substituierten 1-Hydroxyethyl-triazolyl-Derivaten der Formel (I) in Anspruch 1 bzw. von deren Säureadditions-Salzen oder Metallsalz-Komplexen zur Bekämpfung von Pilzen.

8. Verwendung von substituierten 1-Hydroxyethyl-triazolyl-Derivaten der Formel (I) in Anspruch 1 bzw. von deren Säureadditions-Salzen oder Metallsalz-Komplexen als Pflanzenschutzmittel.

9. Verfahren zur Herstellung von fungiziden Mitteln, dadurch gekennzeichnet, daß man substituierte 1-Hydroxyethyl-triazolyl-Derivate der Formel (I) in Anspruch 1 bzw. deren Säureadditions-Salze oder Metallsalz-Komplexe mit Streckmitteln und/oder oberflächenaktiven Mitteln vermischt.

10. Oxirane der Formel

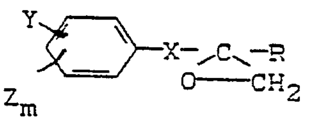

(II)

in welcher R, X, Y, Z und m die in Anspruch 1 angegebene Bedeutung besitzen.

## Claims

1. Substituted 1-hydroxyethyl-triazolyl derivatives of the general formula

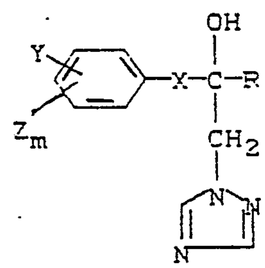

(I)

in which

R represents straight-chain or branched alkyl with 1 to 4 carbon atoms, cycloalkyl which has 3 to 7 carbon atoms and is optionally mono- or polysubstituted by identical or different substituents, the substituents which may be mentioned being : halogen, alkyl with 1 to 4 carbon atoms and alkoxy with 1 to 2 carbon atoms, also represents phenyl which is optionally mono- or polysubstituted by identical or different substituents, possible substituents being the phenyl substituents mentioned under $R^3$, and represents the groupings

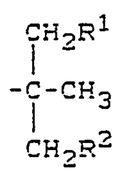

and

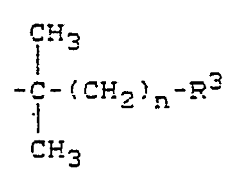

$R^1$ represents hydrogen or halogen ;

$R^2$ represents halogen ;

$R^3$ represents alkyl, alkoxy and alkylthio with in each case 1 to 4 carbon atoms, halogenoalkoxy and halogenoalkylthio with in each case 1 to 2 carbon atoms and 1 to 5 identical or different halogen atoms, also represents alkenyl with 2 to 6 carbon atoms, alkoxycarbonyl with 1 to 4 carbon atoms in the alkyl part, cyano, and phenyl, phenoxy, phenylthio, phenylalkoxy with 1 to 4 carbon atoms in the alkyl part and phenylalkylthio with 1 to 4 carbon atoms in the alkyl part, each of which is optionally mono- or polysubstituted, the phenyl substituents which may be mentioned in each case being : halogen, alkyl with 1 to 4 carbon atoms ; alkoxy and alkylthio with in each case 1 to 2 carbon atoms ; halogenoalkyl, halogenoalkoxy and halogenoalkylthio with in each case 1 to 2 carbon and 1 to 5 identical or different halogen atoms, cyclohexyl, dialkylamino with 1 to 4 carbon atoms in each alkyl part, nitro, cyano, alkoxycarbonyl with 1 to 4 carbon atoms in the alkyl part, and phenyl which is optionally substituted by halogen,

n represents the numbers 0, 1 or 2 ;

X represents the groupings $—OCH_2—$, $—SCH_2—$, $—(CH_2)_p—$ or $—CH = CH—$ ;

Y represents the groupings $—CO—Y^1$, $—C(OR^4)_2—Y^1$ and

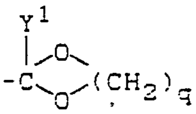

and also the grouping $-C(Y^1) = N-OY^2$ ;

$Y^1$ represents hydrogen, alkyl with 1 to 4 carbon atoms, alkenyl and alkinyl with in each case 2 to 4 carbon atoms, cycloalkyl which has 3 to 7 carbon atoms and is optionally mono- or polysubstituted by identical or different substituents, the substituents which may be mentioned being : halogen, alkyl with 1 to 4 carbon atoms and alkoxy with 1 to 2 carbon atoms, and represents phenyl or benzyl, each of which is optionally mono- or polysubstituted by identical or different substituents, possible substituents being the phenyl substituents mentioned under $R^3$ ;

$Y^2$ represents hydrogen, straight-chain or branched alkyl with 1 to 4 carbon atoms, alkenyl and alkinyl with in each case 2 to 6 carbon atoms, cycloalkyl which has 3 to 7 carbon atoms and is optionally mono- or polysubstituted by identical or different substituents, the substituents which may be mentioned being : halogen, alkyl with 1 to 4 carbon atoms and alkoxy with 1 to 2 carbon atoms ; and represents benzyl which is optionally mono- or polysubstituted by identical or different substituents, possible substituents being the phenyl substituents mentioned under $R^3$ ;

$R^4$ represents alkyl with 1 to 4 carbon atoms ;

q represents the numbers 2 or 3 ;

Z represents halogen, alkyl, alkoxy and alkylthio with in each case 1 to 4 carbon atoms, and halogenalkyl, halogenoalkoxy or halogenoalkylthio with in each case 1 to 2 carbon atoms and 1 to 5 identical or different halogen atoms ;

m represents the numbers 0, 1 or 2 and

p represents the numbers 0, 1 or 2 ;

and their acid addition salts and metal salt complexes.

2. Compounds of the general formula (I) in Claim 1, wherein

R represents tert.-butyl or isopropyl, represents cyclopropyl, cyclopentyl or cyclohexyl, each of which is optionally mono- to trisubstituted by identical or different substituents, the substituents which may be mentioned being : methyl, ethyl, isopropyl, methoxy and ethoxy, also represents phenyl which is optionally mono- to trisubstituted by identical or different substituents from amongst fluorine, chlorine, methyl, trifluoromethyl, phenyl and chlorophenyl, and represents the groupings

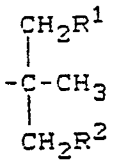 and 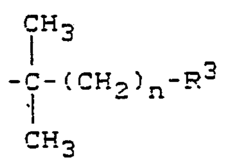

wherein

$R^1$ represents hydrogen, fluorine or chlorine ;

$R^2$ represents fluorine or chlorine ;

$R^3$ represents methyl, ethyl, propyl, methoxy, ethoxy, methylthio, ethylthio, trifluoromethoxy, trifluoromethylthio, vinyl, methoxycarbonyl, ethoxycarbonyl, cyano, and phenyl, phenoxy, phenylthio, phenylmethoxy or phenylmethylthio, each of which is optionally mono- to disubstituted by identical or different substituents, it being possible for the last five radicals mentioned to be substituted by fluorine, chlorine, methyl, ethyl, methoxy, methylthio, trifluoromethyl, trifluoromethoxy, trifluoromethylthio, dimethylamino, methoxycarbonyl and ethoxycarbonyl ; and

n represents the numbers 0, 1 or 2 ;

X represents the groupings $-OCH_2-$, $-SCH_2-$, $-(CH_2)_p-$ or $-CH = CH-$ ;

Y represents the groupings $-CO-Y^1$, $-C(OR^4)_2-Y^1$ and

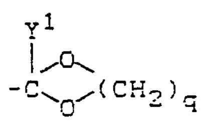

and also the grouping $-C(Y^1) = N-OY^2$ ;

$Y^1$ represents hydrogen, methyl, ethyl, isopropyl or phenyl which is optionally mono- to disubstituted by identical or different substituents from amongst fluorine, chlorine methyl or trifluoromethyl ;

$Y^2$ represents hydrogen, methyl, ethyl, n-propyl, n-butyl, allyl, propargyl or benzyl which is optionally mono- to disubstituted by identical or different substituents from amongst fluorine, chlorine, methyl, trifluoromethyl or trifluoromethoxy ;

$R^4$ represents methyl, ethyl or propyl ; and

q represents the numbers 2 or 3 ;

Z represents fluorine, chlorine, bromine, methyl, methoxy, methylthio, trifluoromethyl, trifluoromethoxy or trifluoromethylthio, and

m represents the numbers 0, 1 or 2 ; and

p represents the numbers 0, 1 or 2.

3. Process for the preparation of substituted 1-hydroxyethyltriazolyl derivatives of the general formula

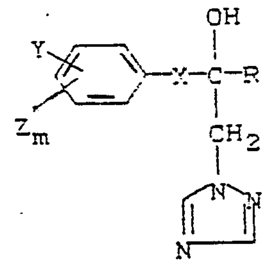

(I)

in which

R represents straight-chain or branched alkyl with 1 to 4 carbon atoms, cycloalkyl which has 3 to 7 carbon atoms and is optionally mono- or polysubstituted by identical or different substituents, the substituents which may be mentioned being : halogen, alkyl with 1 to 4 carbon atoms and alkoxy with 1 to 2 carbon atoms, also represents phenyl which is optionally mono- or polysubstituted by identical or different substituents, possible substituents being the phenyl substituents mentioned under $R^3$, and represents the groupings

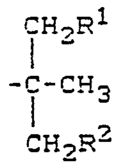

and

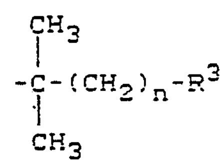

$R^1$ represents hydrogen or halogen ;

$R^2$ represents halogen ;

$R^3$ represents alkyl, alkoxy and alkylthio with in each case 1 to 4 carbon atoms, halogenoalkoxy and halogenoalkylthio with in each case 1 to 2 carbon atoms and 1 to 5 identical or different halogen atoms, also represents alkenyl with 2 to 6 carbon atoms, alkoxycarbonyl with 1 to 4 carbon atoms in the alkyl part, cyano, and phenyl, phenoxy, phenylthio, phenylalkoxy with 1 to 4 carbon atoms in the alkyl part and phenylalkylthio with 1 to 4 carbon atoms in the alkyl part, each of which is optionally mono- or polysubstituted, the phenyl substituents which may be mentioned in each case being : halogen, alkyl with 1 to 4 carbon atoms ; alkoxy and alkylthio with in each case 1 to 2 carbon atoms ; halogenoalkyl, halogenoalkoxy and halogenoalkylthio with in each case 1 to 2 carbon and 1 to 5 identical or different halogen atoms, cyclohexyl, dialkylamino with 1 to 4 carbon atoms in each alkyl part, nitro, cyano, alkoxycarbonyl with 1 to 4 carbon atoms in the alkyl part, and phenyl which is optionally substituted by halogen,

n represents the numbers 0, 1 or 2 ;

X represents the groupings $—OCH_2—$, $—SCH_2—$, $—(CH_2)_p—$ or $—CH = CH—$ ;

Y represents the groupings $—CO—Y^1$, $—C(OR^4)_2—Y^1$ and

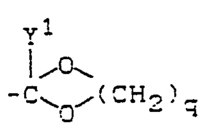

and also the grouping $—C(Y^1) = N—OY^2$ ;

$Y^1$ represents hydrogen, alkyl with 1 to 4 carbon atoms, alkenyl and alkinyl with in each case 2 to 4 carbon atoms, cycloalkyl which has 3 to 7 carbon atoms and is optionally mono- or polysubstituted by identical or different substituents, the substituents which may be mentioned being : halogen, alkyl with 1 to 4 carbon atoms and alkoxy with 1 to 2 carbon atoms, and represents phenyl or benzyl, each of which is optionally mono- or polysubstituted by identical or different substituents, possible substituents being the phenyl substituents mentioned under $R^3$ ;

$Y^2$ represents hydrogen, straight-chain or branched alkyl with 1 to 4 carbon atoms, alkenyl and alkinyl with in each case 2 to 6 carbon atoms, cycloalkyl which has 3 to 7 carbon atoms and is optionally mono- or polysubstituted by identical or different substituents, the substituents which may be mentioned being : halogen, alkyl with 1 to 4 carbon atoms and alkoxy with 1 to 2 carbon atoms ; and represents benzyl which is optionally mono- or polysubstituted by identical or different substituents, possible

substituents being the phenyl substituents mentioned under $R^3$;

$R^4$ represents alkyl with 1 to 4 carbon atoms;

q represents the numbers 2 or 3;

Z represents halogen, alkyl, alkoxy and alkylthio with in each case 1 to 4 carbon atoms, and halogenoalkyl, halogenoalkoxy or halogenalkylthio with in each case 1 to 2 carbon atoms and 1 to 5 identical or different halogen atoms;

m represents the numbers 0, 1 or 2 and

p represents the numbers 0, 1 or 2;

and their acid addition salts and metal salt complexes, characterised in that

a) oxiranes of the formula

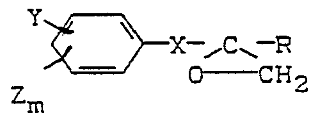

(II)

in which R, X, Y, Z and m have the abovementioned meaning, are reacted with 1,2,4-triazole of the formula

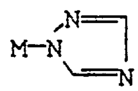

(III)

in which M represents hydrogen or an alkali metal, in the presence of a diluent and, if appropriate, in the presence of a base, or

b) triazolylmethyl oxiranes of the formula

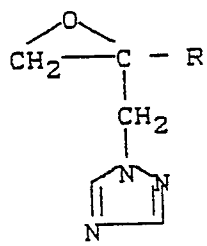

(IV)

in which R has the abovementioned meaning, are reacted with (thio)phenols of the formula

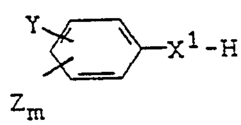

(V)

in which

Y, Z and m have the abovementioned meaning and

$X^1$ represents oxygen or sulphur,

in the presence of a diluent and, if appropriate, in the presence of a base, or

c) the compounds according to the invention which are obtainable by processes (a) and (b) and have the formula

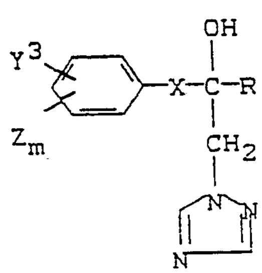

(Ia)

in which

R, X, Z and m have the abovementioned meaning and

$Y^3$ represents the groupings $—CO—Y^1$, $—C(OR^4)_2Y^1$ and

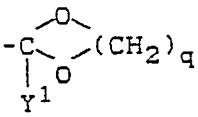

are reacted with hydroxylamine derivatives of the formula

$$H_2N—O—Y^2 \qquad (VI)$$

in which $Y^2$ has the abovementioned meaning, in the presence of a diluent, or

d) the compounds according to the invention which are obtainable by processes a), b) and c) and have the formula

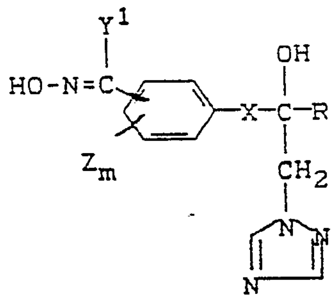

(Ib)

in which R, X, $Y^1$, Z and m have the abovementioned meaning, are reacted with halides of the formula

$$Y^4—Hal \qquad (VII)$$

in which

Hal represents chlorine, bromine or iodine and

$Y^4$ represents the meanings of $Y^2$, except hydrogen,

in the presence of a diluent and, if appropriate, in the presence of a base, and, if desired, an acid or a metal salt is then added on to the compounds of the formula (I) obtained by the above processes.

4. Plant protection agents, characterised in that they contain at least one substituted 1-hydroxyethyl-triazolyl derivative of the formula (I) in Claim 1 or an acid addition salt or metal salt complex of a compound of the formula (I).

5. Fungicidal agents, characterised in that they contain at least one substituted 1-hydroxyethyl-triazolyl derivative of the formula (I) in Claim 1 or an acid addition salt or metal salt complex of a compound of the formula (I).

6. Method of combating fungi, characterised in that substituted 1-hydroxyethyl-triazolyl derivatives of the formula (I) in Claim 1 or their acid addition salts or metal salt complexes are allowed to act on fungi or their environment.

7. Use of substituted 1-hydroxyethyl-triazolyl derivatives of the formula (I) in Claim 1 or their acid addition salts or metal salt complexes for combating fungi.

8. Use of substituted 1-hydroxyethyl-triazolyl derivatives of the formula (I) in Claim 1 or their acid addition salts or metal salt complexes, as plant protection agents.

9. Process for the preparation of fungicidal agents, characterised in that substituted 1-hydroxyethyl-triazolyl derivatives of the formula (I) in Claim 1 or their acid addition salts or metal salt complexes are mixed with extenders and/or surface-active agents.

10. Oxiranes of the formula

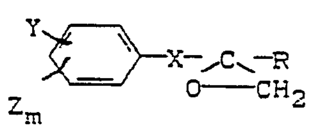

(II)

in which R, X, Y, Z and m have the meaning given in Claim 1.

**Revendications**

1. Dérivés 1-hydroxyéthyl-triazolyliques substitués de formule générale

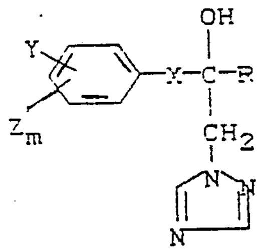

(I)

dans laquelle

R désigne un groupe alkyle à chaîne droite ou ramifiée ayant 1 à 4 atomes de carbone, un groupe cycloalkyle de 3 à 7 atomes de carbone portant éventuellement un ou plusieurs substituants identiques ou différents, et on mentionne alors comme substituants : un halogène, un reste alkyle ayant 1 à 4 atomes de carbone et un reste alkoxy ayant 1 ou 2 atomes de carbone, en outre un groupe phényle portant éventuellement un ou plusieurs substituants identiques ou différents, et on considère alors comme substituants les substituants du groupe phényle mentionnés pour $R^3$, ainsi que les groupements

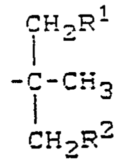

et

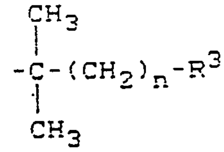

$R^1$ désigne l'hydrogène ou un halogène ;

$R^2$ est un halogène ;

$R^3$ désigne des groupes alkyle, alkoxy et alkylthio ayant chacun 1 à 4 atomes de carbone, des groupes halogénalkoxy et halogénalkylthio ayant chacun 1 ou 2 atomes de carbone et 1 à 5 atomes d'halogènes identiques ou différents, en outre un groupe alcényle ayant 2 à 6 atomes de carbone, un groupe alkoxycarbonyle ayant 1 à 4 atomes de carbone dans la partie alkyle, un groupe cyano ainsi qu'un groupe phényle, phénoxy, phénylthio, phénylalkoxy portant éventuellement un ou plusieurs substituants et ayant 1 à 4 atomes de carbone dans la partie alkyle, et un groupe phénylalkylthio ayant 1 à 4 atomes de carbone dans la partie alkyle, et on mentionne alors comme substituants du groupe phényle dans chaque cas : un halogène, un reste alkyle ayant 1 à 4 atomes de carbone ; un reste alkoxy et un reste alkylthio ayant chacun 1 ou 2 atomes de carbone ; un reste halogénalkyle, un reste halogénalkoxy et un reste halogénalkylthio ayant chacun 1 ou 2 atomes de carbone et 1 à 5 atomes d'halogènes identiques ou différents, cyclohexyle, dialkylamino ayant 1 à 4 atomes de carbone dans chaque partie alkyle, nitro, cyano, alkoxycarbonyle ayant 1 à 4 atomes de carbone dans la partie alkyle, ainsi qu'un groupe phényle éventuellement substitué par un halogène,

n représente les nombres 0, 1 ou 2 ;

X représente les groupements $—OCH_2—$, $—SCH_2—$, $—(CH_2)_p—$ ou $—CH = CH—$ ;

Y représente les groupements $—CO—Y^1$, $—C(OR^4)_2—Y^1$ et

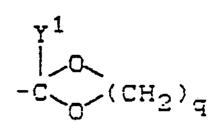

ainsi que le groupement $—C(Y^1) = N—OY^2$ ;

$Y^1$ représente l'hydrogène, un groupe alkyle ayant 1 à 4 atomes de carbone, un groupe alcényle et un groupe alcynyle ayant chacun 2 à 4 atomes de carbone, un groupe cycloalkyle de 3 à 7 atomes de carbone portant éventuellement un ou plusieurs substituants identiques ou différents, et on mentionne alors comme substituants : un halogène, un reste alkyle ayant 1 à 4 atomes de carbone et un reste alkoxy ayant 1 ou 2 atomes de carbone, ainsi qu'un reste phényle ou benzyle dont chacun porte éventuellement un ou plusieurs substituants identiques ou différents, et on considère alors comme substituants les substituants du groupe phényle mentionnés pour $R^3$ ;

$Y^2$ désigne l'hydrogène, un groupe alkyle à chaîne droite ou ramifiée ayant 1 à 4 atomes de carbone, un groupe alcényle et un groupe alcynyle ayant chacun 2 à 6 atomes de carbone, un groupe cycloalkyle de 3 à 7 atomes de carbone portant éventuellement un ou plusieurs substituants identiques ou différents, et on mentionne alors comme substituants : un halogène, un groupe alkyle ayant 1 à 4 atomes de carbone et un groupe alkoxy ayant 1 ou 2 atomes de carbone ; ainsi qu'un groupe benzyle portant éventuellement un ou plusieurs substituants identiques ou différents, et on considère alors comme substituants les substituants du groupe phényle mentionnés pour $R^3$ ;

$R^4$ est un groupe alkyle ayant 1 à 4 atomes de carbone ;

q représente les nombres 2 ou 3 ;

Z est un halogène, un groupe alkyle, un groupe alkoxy et un groupe alkylthio ayant chacun 1 à

4 atomes de carbone, ainsi qu'un groupe halogénalkyle, halogénalkoxy ou halogénalkylthio ayant dans chaque cas 1 ou 2 atomes de carbone et 1 à 5 atomes d'halogènes identiques ou différents ;

m représente les nombres 0, 1 ou 2 et

p représente les nombres 0, 1 ou 2 ;

ainsi que leurs sels d'addition d'acides et leurs complexes de sels métalliques.

2. Composés de formule générale (I) suivant la revendication 1, dans laquelle

R est un groupe tertio-butyle ou isopropyle, un groupe cyclopropyle, cyclopentyle ou cyclohexyle dont chacun porte éventuellement un à trois substituants identiques ou différents, et on mentionne alors comme substituants : un radical méthyle, éthyle, isopropyle, méthoxy et éthoxy, en outre un groupe phényle portant éventuellement un à trois substituants identiques ou différents choisis entre fluor, chlore, méthyle, trifluorométhyle, phényle et chlorophényle, de même que les groupements

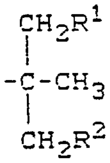

et

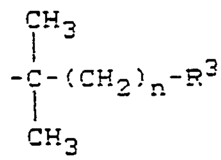

où

$R^1$ représente l'hydrogène, le fluor ou le chlore ;

$R^2$ est le fluor ou le chlore ;

$R^3$ est un groupe méthyle, éthyle, propyle, méthoxy, éthoxy, méthylthio, éthylthio, trifluorométhoxy, trifluorométhylthio, vinyle, méthoxycarbonyle, éthoxycarbonyle, cyano ainsi qu'un groupe phényle, phénoxy, phénylthio, phénylméthoxy ou phénylméthylthio, chacun portant éventuellement un ou deux substituants identiques ou différents, les cinq restes mentionnés en dernier lieu pouvant être substitués par du fluor, du chlore, les radicaux méthyle, éthyle, méthoxy, méthylthio, trifluorométhyle, trifluorométhoxy, trifluorométhylthio, diméthylamino, méthoxycarbonyle et éthoxycarbonyle ; et

n représente les nombres 0, 1 ou 2 ;

X représente les groupements $—OCH_2—$, $—SCH_2—$, $—(CH_2)_p—$ ou $—CH = CH—$ ;

Y représente les groupements $—CO—Y^1$, $—C(OR^4)_2—Y^1$ et

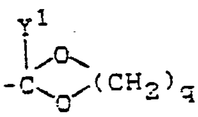

ainsi que le groupement $—C(Y^1) = N—OY^2$ ;

$Y^1$ représente l'hydrogène, un groupe méthyle, éthyle, isopropyle ou un groupe phényle portant éventuellement un ou deux substituants identiques ou différents tels que fluor, chlore, méthyle ou trifluorométhyle ;

$Y^2$ représente l'hydrogène, un groupe méthyle, éthyle, n-propyle, n-butyle, allyle, propargyle ou un groupe benzyle portant éventuellement un ou deux substituants identiques ou différents choisis entre fluor, chlore, méthyle, trifluorométhyle ou trifluorométhoxy ;

$R^4$ est un groupe méthyle, éthyle ou propyle ; et

q représente les nombres 2 ou 3 ;

Z désigne le fluor, le chlore, le brome, un groupe méthyle, méthoxy, méthylthio, trifluorométhyle, trifluorométhoxy ou trifluorométhylthio, et

m représente les nombres 0, 1 ou 2 ; et

p représente les nombres 0, 1 ou 2.

3. Procédé de production de dérivés 1-hydroxy-éthyl-triazolyliques substitués de formule générale

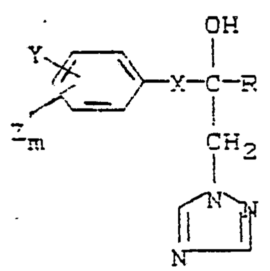

(I)

dans laquelle

R désigne un groupe alkyle à chaîne droite ou ramifiée ayant 1 à 4 atomes de carbone, un groupe cycloalkyle de 3 à 7 atomes de carbone portant éventuellement un ou plusieurs substituants identiques

ou différents, et on mentionne alors comme substituants : un halogène, un reste alkyle ayant 1 à 4 atomes de carbone et un reste alkoxy ayant 1 ou 2 atomes de carbone, en outre un groupe phényle portant éventuellement un ou plusieurs substituants identiques ou différents, et on considère alors comme substituants les substituants du groupe phényle mentionnés pour $R^3$, ainsi que les groupements

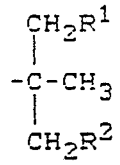 et 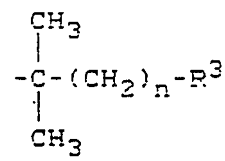

$R^1$ désigne l'hydrogène ou un halogène ;

$R^2$ est un halogène ;

$R^3$ désigne des groupes alkyle, alkoxy et alkylthio ayant chacun 1 à 4 atomes de carbone, des groupes halogénalkoxy et halogénalkylthio ayant chacun 1 ou 2 atomes de carbone et 1 à 5 atomes d'halogènes identiques ou différents, en outre un groupe alcényle ayant 2 à 6 atomes de carbone, un groupe alkoxycarbonyle ayant 1 à 4 atomes de carbone dans la partie alkyle, un groupe cyano ainsi qu'un groupe phényle, phénoxy, phénylthio, phénylalkoxy portant éventuellement un ou plusieurs substituants et ayant 1 à 4 atomes de carbone dans la partie alkyle, et un groupe phénylalkylthio ayant 1 à 4 atomes de carbone dans la partie alkyle, et on mentionne alors comme substituants du groupe phényle dans chaque cas : un halogène, un reste alkyle ayant 1 à 4 atomes de carbone ; un reste alkoxy et un reste alkylthio ayant chacun 1 ou 2 atomes de carbone ; un reste halogénalkyle, un reste halogénalkoxy et un reste halogénalkylthio ayant chacun 1 ou 2 atomes de carbone et 1 à 5 atomes d'halogènes identiques ou différents, cyclohexyle, dialkylamino ayant 1 à 4 atomes de carbone dans chaque partie alkyle, nitro, cyano, alkoxycarbonyle ayant 1 à 4 atomes de carbone dans la partie alkyle, ainsi qu'un groupe phényle éventuellement substitué par un halogène,

n représente les nombres 0, 1 ou 2 ;

X représente les groupements $—OCH_2—$, $—SCH_2—$, $—(CH_2)_p—$ ou $—CH = CH—$ ;

Y représente les groupements $—CO—Y^1$, $—C(OR^4)_2—Y^1$ et

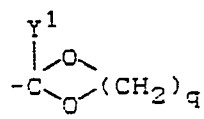

ainsi que le groupement $—C(Y^1) = N—OY^2$ ;

$Y^1$ représente l'hydrogène un groupe alkyle ayant 1 à 4 atomes de carbone, un groupe alcényle et un groupe alcynyle ayant chacun 2 à 4 atomes de carbone, un groupe cycloalkyle de 3 à 7 atomes de carbone portant éventuellement un ou plusieurs substituants identiques ou différents, et on mentionne alors comme substituants : un halogène, un reste alkyle ayant 1 à 4 atomes de carbone et un reste alkoxy ayant 1 ou 2 atomes de carbone, ainsi qu'un reste phényle ou benzyle dont chacun porte éventuellement un ou plusieurs substituants identiques ou différents, et on considère alors comme substituants les substituants du groupe phényle mentionnés pour $R^3$ ;

$Y^2$ désigne l'hydrogène, un groupe alkyle à chaîne droite ou ramifiée ayant 1 à 4 atomes de carbone, un groupe alcényle et un groupe alcynyle ayant chacun 2 à 6 atomes de carbone, un groupe cycloalkyle de 3 à 7 atomes de carbone portant éventuellement un ou plusieurs substituants identiques ou différents, et on mentionne alors comme substituants : un halogène, un groupe alkyle ayant 1 à 4 atomes de carbone et un groupe alkoxy ayant 1 ou 2 atomes de carbone ; ainsi qu'un groupe benzyle portant éventuellement un ou plusieurs substituants identiques ou différents, et on considère alors comme substituants les substituants du groupe phényle mentionnés pour $R^3$ ;

$R^4$ est un groupe alkyle ayant 1 à 4 atomes de carbone ;

q représente les nombres 2 ou 3 ;

Z est un halogène, un groupe alkyle, un groupe alkoxy et un groupe alkylthio ayant chacun 1 à 4 atomes de carbone, ainsi qu'un groupe halogénalkyle, halogénalkoxy ou halogénalkylthio ayant dans chaque cas 1 ou 2 atomes de carbone et 1 à 5 atomes d'halogènes identiques ou différents ;

m représente les nombres 0, 1 ou 2 et

p représente les nombres 0, 1 ou 2

ainsi que de leurs sels d'addition d'acides et de leurs complexes de sels métalliques, caractérisé en ce que :

a) on fait réagir des oxiranes de formule

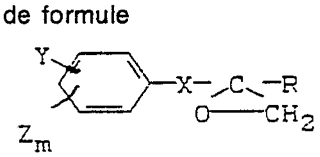 (II)

dans laquelle R, X, Y, Z et m ont la définition indiquée ci-dessus, avec le 1,2,4-triazole de formule

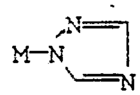

(III)

dans laquelle M représente l'hydrogène ou un métal alcalin, en présence d'un diluant et, le cas échéant, en présence d'une base, ou bien

b) on fait réagir des triazolylméthyl-oxiranes de formule

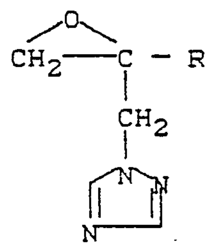

(IV)

dans laquelle R a la définition indiquée ci-dessus, avec des (thio)phénols de formule

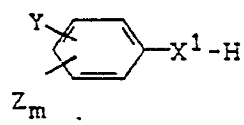

(V)

dans laquelle
Y, Z et m ont la définition indiquée ci-dessus, et
$X^1$ représente l'oxygène ou le soufre,
en présence d'un diluant et, le cas échéant, en présence d'une base, ou bien

c) on fait réagir les composés de l'invention pouvant être obtenus par les procédés (a) et (b), de formule

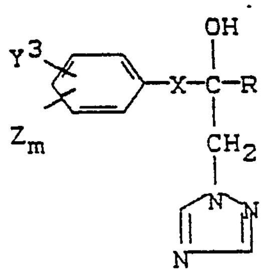

(Ia)

dans laquelle
R, X, Z et m ont la définition indiquée ci-dessus et
$Y^3$ représente les groupements $—CO—Y^1$, $—C(OR^4)_2Y^1$ et

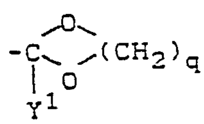

avec des dérivés d'hydroxylamine de formule

$$H_2N—O—Y^2 \qquad \text{(IV)}$$

dans laquelle $Y^2$ a la définition indiquée ci-dessus, en présence d'un diluant, ou bien

d) on fait réagir les composés de l'invention pouvant être obtenus par les procédés a), b) et c), de formule

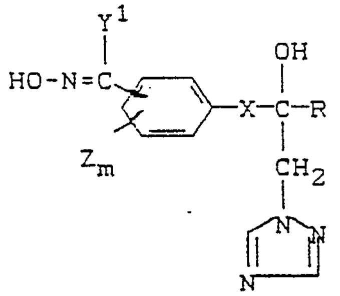

(Ib)

dans laquelle R, X, $Y^1$, Z et m ont la définition indiquée ci-dessus, avec des halogénures de formule

$Y^4$—Hal (VII)

dans laquelle

Hal représente le chlore, le brome ou l'iode et

$Y^4$ a les définitions de $Y^2$, excepté l'hydrogène, en présence d'un diluant et, le cas échéant, en présence d'une base,

et on additionne ensuite éventuellement un acide ou un sel métallique sur les composés de formule (I) pouvant être obtenus par les procédés ci-dessus.

4. Compositions phytosanitaires, caractérisées par une teneur en au moins un dérivé 1-hydroxyéthyl-triazolylique substitué de formule (I) suivant la revendication 1 ou en un sel d'addition d'acide ou en un complexe de sel métallique d'un composé de formule (I).

5. Compositions fongicides, caractérisées par une teneur en au moins un dérivé 1-hydroxyéthyltriazolylique substitué de formule (I) suivant la revendication 1 ou un sel d'addition d'acide ou un complexe de sel métallique d'un composé de formule (I).

6. Procédé pour combattre des champignons, caractérisé en ce qu'on fait agir des dérivés 1-hydroxyéthyl-triazolyliques substitués de formule (I) suivant la revendication 1 ou leurs sels d'addition d'acides ou leurs complexes de sels métalliques sur des champignons ou sur leur milieu.

7. Utilisation de dérivés 1-hydroxyéthyltriazolyliques substitués de formule (I) suivant la revendication 1 et de leurs sels d'addition d'acides ou de leurs complexes de sels métalliques pour combattre des champignons.

8. Utilisation de dérivés 1-hydroxyéthyltriazolyliques substitués de formule (I) suivant la revendication 1 ou de leurs sels d'addition d'acides ou de leurs complexes de sels métalliques comme agents phytosanitaires.

9. Procédé de préparation de compositions fongicides, caractérisé en ce qu'on mélange des dérivés 1-hydroxyéthyl-triazolyliques substitués de formule (I) suivant la revendication 1 ou leurs sels d'addition d'acides ou leurs complexes de sels métalliques avec des diluants et/ou des agents tensio-actifs.

10. Oxiranes de formule

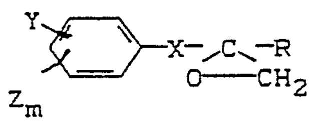

(II)

dans laquelle R, X, Y, Z et m ont la définition indiquée dans la revendication 1.